# EUROPEAN PATENT SPECIFICATION

(11) **EP 1 806 142 B1**
(45) Date of publication and mention of the grant of the patent: **14.07.2010**
(21) Application number: 06025571.8
(22) Date of filing: 02.10.1998
(51) Int. Cl.: A61K 38/17, A61K 39/295, C07K 14/02, C07K 14/025, C07K 16/08, C07K 16/18, C12N 15/62, C12Q 1/68, G01N 33/50

(54) **Method for the production of (poly)peptides by using truncated variants of the SV 40 large T antigen with an intact N terminus**
Methode zur Herstellung von (Poly)peptiden unter Verwendung von verkürzten Varianten von SV40 Large T antigen mit einem intakten N Terminus
Procédé de production de (poly)peptides à l'aide de variants tronqués de SV40 large T antigen ayant une terminaison N intacte

(43) Date of publication of application: 11.07.2007
(62) Divisional of application: 98950105.1
(73) Proprietor: Rhein Biotech Gesellschaft für neue biotechnologische Prozesse und Produkte mbH, 40595 Düsseldorf (DE)
(72) Inventor: Reimann, Hansjörg, 89081 Ulm (DE); Schirmbeck, Reinhold, 86381 Krumbach (DE)
(74) Representative: Herzog, Martin

(56) References cited:
- THOMAS JEFFREY G ET AL: "Protein misfolding and inclusion body formation in recombinant Escherichia coli cells overexpressing heat-shock proteins" JOURNAL OF BIOLOGICAL CHEMISTRY, AMERICAN SOCIETY OF BIOLOCHEMICAL BIOLOGISTS, BIRMINGHAM,, US, vol. 271, no. 19, 1996, pages 11141-11147, XP002193565 ISSN: 0021-9258
- SCHIRMBECK R ET AL: "Stress protein ( hsp73 )-mediated, TAP-independent processing of endogenous, truncated SV40 large T antigen for Db-restricted peptide presentation" EUROPEAN JOURNAL OF IMMUNOLOGY, WEINHEIM, DE, vol. 27, no. 8, August 1997 (1997-08), pages 2016-2023, XP002107333 ISSN: 0014-2980
- SCHIRMBECK R ET AL: "Peptide transporter-independent, stress protein-mediated endosomal processing of endogenous protein antigens for major histocompatibility complex class I presentation" EUROPEAN JOURNAL OF IMMUNOLOGY, WEINHEIM, DE, vol. 24, no. 7, July 1994 (1994-07), pages 1478-1486, XP002107334 ISSN: 0014-2980
- SCHIRMBECK R ET AL: "Truncated or chimeric endogenous protein antigens gain immunogenicity for B cells by stress protein-facilitated expression" EUROPEAN JOURNAL OF IMMUNOLOGY, WEINHEIM, DE, vol. 29, no. 5, May 1999 (1999-05), pages 1740-1749, XP002107329 ISSN: 0014-2980

## Description

The present invention relates to a polynucleotide encoding a fusion protein which is stable in a cell, said fusion protein comprising a first (poly)peptide and a second (poly)peptide which co-precipitates a chaperone. The present invention also relates to a vector comprising the polynucleotide of the invention, a host cell comprising the polynucleotide or the vector of the invention, and a method for the production of the fusion protein of the invention. Also described are methods for the production of said first (poly)peptide, of a fusion protein/chaperone complex, and of an antibody directed against said first (poly)peptide, as well as a method of immunizing a subject with the polynucleotide, the vector, the fusion protein, said first (poly)peptide and/or said fusion protein/chaperone complex of the invention. In addition, the present invention relates to a kit and a diagnostic composition comprising the polynucleotide, the vector, the host cell, the fusion protein, the first (poly)peptide, the fusion protein/chaperone complex and/or the antibody of the invention. The present invention, furthermore, relates to a method for the detection of the presence of an epitope comprised in a (poly)peptide. Additionally described is a pharmaceutical composition comprising the polynucleotide, the vector, the fusion protein, the first (poly)peptide, the antibody, and/or the complex of the present invention and, optionally, a pharmaceutically acceptable carrier and/or diluent, said pharmaceutical composition being preferably a vaccine. Finally, the present invention relates to the use of the polynucleotide or the vector of the invention for the production of an antibody directed against said first (poly)peptide, and the use of a (poly)peptide comprising an epitope detected by the method of the present invention or a complex produced by the method of the invention for the production of an antibody.

Chaperones play essential roles in cells under normal conditions. They assist folding of nascent proteins, they guide translocation of proteins through membranes, they hold subunits in an assembly-competent state, they disassemble oligomeric protein complexes, they solubilize denatured protein aggregates, and they facilitate proteolytic degradation of mutant or irreversibly damaged proteins in degradative organelles or proteasomes (reviewed in Bukau, B. and A.L. Horwich, *Cell* 92 (1998):351). There exist different classes of chaperones (originally called heat shock proteins (Hsp) due to their increased abundance following heat shock) the best studied of which are the ubiquitous Hsp70 and Hsp60 families. The C-terminal domains of Hsp70 and Hsp60 class molecules interact with substrate proteins and immunogenic peptides captured in the cytosol and in the endoplasmic reticulum, and peptides can be eluted *in vitro* from both classes of molecules (Blachere, N.E., Z. Li, R.Y. Chandawarkar, R. Suto, N.S. Jaikaria, S. Basu, H. Udono, and P.K. Srivastava, J.Exp.Med. 186 (1997):1315; Heikema, A., E. Agsteribbe, J. Wilschut, and A. Huckriede, Immunol.Lett. 57 (1997):69; Lammert, E., D. Arnold, M. Nijenhuis, F. Momburg, G.J. Hammerling, J. Brunner, S. Stevanovic, H.G. Rammensee, and H. Schild, Eur.J.Immunol. 27 (1997):923; Peng, P., A. Menoret, and P.K. Srivastava, J.Immunol.Methods 204 (1997):13; Roman, E. and C. Moreno, Immunology 90 (1997):52; Nieland, T.J.F., M.C.A. Tan, M.M. van Muijen, F. Koning, A.M. Kruisbeek, and G.M. van Bleek, Proc.Natl.Acad.Sci.USA 93 (1996):6135; Roman, E. and C. Moreno, Immunology 88 (1996):487; Arnold, D., S. Faath, H.G. Rammensee, and H. Schild, J.Exp.Med. 182 (1995):885; Srivastava, P.K., H. Udono, N.E. Blachere, and Z. U, Immunogenetics 39 (1994):93; Udono, H. and P.K. Srivastava, J.Exp.Med. 178 (1993):1391; Ciupitu, A.-M., M. Petersson, C.L. O'Donnell, K. Williams, S. Jindal, R. Kiessling, and M.J. Welsh, J.Exp.Med.187 (1998):685).
Hsp73 is a prominent, constitutively expressed, cytosolic stress protein of the Hsp70 family. Among many other known functions, this Hsp mediates selective degradation of cytosolic proteins in an endolysosomal compartment (reviewed in Dice, J.F. and S.R. Terlecky. 1994. Selective degradation of cytosolic proteins by lysosomes. In Cellular proteolytic systems. A.J. Clechanover and A.L. Schwartz, editors. Wiley-Liss, 55-64; Hayes, S.A. and J.F. Dice, J.Cell Biol. 132 (1996):255). It specifically recognizes KFERQ-like peptide sequences and targets the captured proteins for lysosomal degradation in response to various stimuli (Chiang, H.-L., S.R. Terlecky, C.P. Plant, and J.F. Dice, Science 246 (1989):382; Dice, J.F., Trends Biochem.Sci. 15 (1990):305; Terlecky, S.R., H.-L. Chiang, T.S. Olson, and J.F. Dice, J.Biol.Chem. 267 (1992):9202). Hsp73-associated proteins directly cross the membrane bilayer to enter lysosomes (Dice, J.F. and S.R. Terlecky. 1994. Selective degradation of cytosolic proteins by lysosomes. In Cellular proteolytic systems. A.J. Ciechanover and A.L. Schwartz, editors. Wiley-Liss, 55-64; Terlecky, S.R., H.-L. Chiang, T.S. Olson, and J.F. Dice, J.Biol.Chem. 267 (1992):9202). The transport of Hsp73-associated proteins into lysosomes has been reconstituted *in vitro* using highly purified lysosomes. Uptake is stimulated by ATP, requires the stress protein Hsp73, is selective and saturable, and does not involve a vesicular pathway. The lysosomal membrane glycoprotein LGP96 has been identified as a receptor for the import of proteins into lysosomes (Terlecky, S.R., H.-L. Chiang, T.S. Olson, and J.F. Dice, J.Biol.Chem. 267 (1992):9202; Cuervo, A.M. and J.F. Dice, Science 273 (1996):501). Hsp73 mediates protein import not exclusively into lysosomes but also into endosomal compartments.
Injection of Hsp/peptide complexes without adjuvants elicits delayed type hypersensitivity reactions, CD8⁺ cytotoxic T lymphocyte (CTL) responses, T cell-mediated tumor rejection, and CTL-dependent protection against lethal virus infection (Blachere, N.E., Z. Li, R.Y. Chandawarkar, R. Suto, N.S. Jaikaria, S. Basu, H. Udono, and P.K. Srivastava, J.Exp.Med. 186 (1997):1315; Heikema, A., E. Agsteribbe, J. Wilschut, and A. Huckriede, Immunol.Lett. 57 (1997):69; Roman, E. and C. Moreno, Immunology 90 (1997):52; Roman, E. and C. Moreno, Immunology 88 (1996):487; Arnold, D., S. Faath, H.G. Rammensee, and H. Schild, J.Exp.Med. 182 (1995):885; Udono, H. and P.K. Srivastava, J.Exp.Med. 178 (1993):1391; Ciupltu, A.-M., M. Petersson, C.L. O'Donnell, K. Williams, S. Jindal, R. Kiessling, and M.J. Welsh, J.Exp.Med. 187 (1998):685; Udono, H. and P.K Srivastava, J.Immunol. 152 (1994):5398; Suto, R. and P.K. Srivastava, Science 269 (1995):1585; Suzue, K. and R.A. Young, J.Immunol. 156 (1998):873). Hsp molecules of both bacterial or eukaryotic origin mediate this function. In addition, large protein antigens fused to Hsp show strikingly enhanced immunogenicity (Suzue, K. and RA. Young, J.Immunol. 156 (1996):873).
Alternatively, immunization with plasmid DNA has demonstrated an impressive potential to induce humoral and cellular immune responses to different antigens (reviewed in Donnelly, J.J., J.B. Ulmer, J.W. Shiver, and M.A. Liu, Annu.Rev.Immunol. 15 (1997):617). It has opened new ways to screen for antigenic epitopes and to construct polyvalent vaccines because manipulating recombinant DNA Is much easier than constructing, expressing and purifying chimeric protein antigens. However, the immunogenicity of DNA vaccines depends among other factors on the level of antigen expressed in cells transfected *in vivo* by plasmid DNA inoculation. Evidence suggests that higher antigen concentrations *in situ* are required to prime humoral (B cell) than cellular (T cell) immune responses. This often leads to the effect that endogenous antigens prime MHC-I-restricted T cell responses but no antibody responses.
The above phenomenon is especially true for mutated or truncated proteins which are rapidly cleared from the cytosol of eukaryotic cells by potent proteolytic systems. These proteolytic systems often prevent the expression of such protein fragments by conventional expression systems to steady state levels which are necessary to induce humoral immune responses. However, apart from the apparent need of a certain "threshold" amount of expressed antigen, conventional nucleic acid vaccination approaches also encounter difficulties in inducing humoral immune responses when the antigen to be expressed is, e.g., a strictly intracellular protein.
On the other hand, of course, the above mentioned proteolytic systems render conventional expression systems ineffective to produce such mutated or truncated proteins in amounts necessary to test their functional, structural and/or antigenic properties.
Consequently, it would be highly desirable in the art to have an expression system at hand which allows the synthesis of (poly)peptides such that said (poly)peptides become accessible for investigations as described above and/or for the development of, e.g., novel pharmaceutical compositions, polyvalent nucleic acid vaccines, etc.

Thus, the technical problem underlying the present invention was to solve the above problems of the prior art, namely the provision of an inexpensive and efficient means which reliably allows expression of (poly)peptides within cells.

The solution of the above technical problem is achieved by providing the embodiments characterized in the claims.

Accordingly, the present invention relates to a polynucleotide encoding a fusion protein which is stable in a cell, as defined in claim 1.

As used in accordance with the present invention, the term "stable in a cell" denotes (poly)peptides which have a half-life in a cell of at least two hours. In other words, if the amount of label incorporated into a (poly)peptide in pulse-chase experiments is not reduced to one half within less than two hours after termination of the pulse phase, the corresponding (poly)peptide is regarded stable, (Poly)peptides the half-lives of which are less than two hours or not measurable by pulse-chase experiments at all are regarded "unstable" in accordance with the present invention. Pulse-chase experiments used, e.g., to determine the stability of a compound in a biological system are well known to the person skilled in the art (see, e.g., Damell et al. (eds.), Molecular Cell Biology, 1986, Scientific American Books, Inc., New York; Ausubel et al. (eds.), Current Protocols in Molecular Biology, 1989, Green Publishing Associates and Wiley Interscience, New York). For instance, cells may be incubated in the presence of [³⁵S] methionine for 10 to 15 minutes followed by a chase period of several hours. During said chase period cells may be harvested at different times, proteins of interest isolated, e.g., by immunoprecipitation, and the amount of incorporated label determined, e.g., in SDS-polyacrylamide gel electrophoresis (for a preferred detailed protocol see Examples 2 and 3, infra, and Schirmbeck, R. and J. Reimann, Eur.J.Immunol. 24 (1994):1478). With respect to the stability "in a cell", it will be readily evident for the person skilled in the art that this phrase comprises the stability of a (poly)peptide in any compartment of the cell including, e.g., the cytoplasm, the endoplasmatic reticulum and the compartments involved in the secretory pathway, the nucleus if present, etc.

The term "(poly)peptide." as used in accordance with the present invention encompasses polypeptides or proteins having a length of about 50 to several hundreds of amino acids as well as peptides having a length of about 5 to 50 amino acids.

As used in accordance with the present invention, the phrase "second (poly)peptide which is a viral T-antigen carrying an internal and/or C-terminal deletion which co-precipitates a chaperone belonging to the family of heat shock protein (Hsp)70 chaperones" describes, in general, an interaction of said second (poly)peptide with the chaperone that leads to the precipitation of both the (poly)peptide and the chaperone upon a further (prior or simultaneous) interaction of the (poly)peptide with a precipitating agent. Precipitating agents may be of various nature. However, a prerequisite they must fulfil is that the interaction with said second (poly)peptide is specific. A preferred type of precipitating agent is an antibody such as a monoclonal antibody or a fragment or derivative thereof. Advantageously, the interaction of the (poly)peptide with the chaperone is characterized by a binding constant that is higher than the binding constant between the chaperone and the natural counterpart of said second (poly)peptide within the fusion protein under physiological conditions within a cell. Accordingly, a (poly)peptide which co-precipitates the chaperone interacts with the chaperone in such a way that the interaction originally established during synthesis of said (poly)peptide is maintained after completion of translation and through an (artificial) precipitation step. This allows the co-purification of the chaperone by the precipitation step and the subsequent detection in a suitable detection assay. For example, a (poly)peptide is purified from a cell extract by precipitation with a precipitating agent which specifically binds to said (poly)peptide and which is, optionally, coupled to a carrier. After precipitation, the presence of the isolated (poly)peptide is determined, e.g., by measuring and visualizing radioactive label incorporated previously into said (poly)peptide. Preferably, said (poly)peptide is synthesized in vivo and purified from an extract of cells which were used to express said (poly)peptide. It is further preferred that said precipitating agent is an antibody as mentioned above, that said carrier is protein A-sepharose, and/or that the detection assay comprises SDS-polyacrylamide gel electrophoresis (SDS-PAGE) of the purified (poly)peptide followed by autoradiography of the gel. Of course, it is well known to the person skilled in the art that the detection of the presence of a co-precipitate by a certain assay depends on the sensitivity of said assay but may also be dependent on other parameters which may be varied when performing said assay, In other words, the failure to detect a certain co-precipitate usually does not indicate that said co-precipitate is not present in the analyte. Rather, such a result may mean that the sensitivity of the assay did not suffice and/or the chosen reaction conditions were not appropriate to detect the presence of said co-precipitate. The experimental conditions under which a second (poly)peptide comprised in the fusion protein of the present invention delectably co-precipitates a chaperone are as follows: During a period of about 15 min cells are incubated with a radiolabeled amino acid such that all proteins newly synthesized in this pulse period become radioactively labeled. A subsequent chase with an excess amount of the non-labeled form of this amino acid ensures that only proteins synthesized during this 15 min pulse period are radioactive and detectable by this tag during the subsequent chase period. In accordance with the present invention a detectable co-precipitation of a chaperone still has to occur after 5 min, preferably after 10 min, more preferably after 20 min, and most preferably after 30 min until to about 2 hours, preferably to about 4 hours, and most preferably to about 6 hours after the end of the pulse period/the beginning of the chase period.
As mentioned above, most nascent cellular (poly)peptides are transiently associated with chaperones which assist in the folding of the nascent (poly)peptide and, afterwards, release the properly folded (poly)peptide (Craig, E.A., Science 260 (1993):1902; Welch, W.J. and J.P. Suhan, J.Cell Biol. 103 (1993):2035). Chaperones which are associated in this transient way with (poly)peptides are not detectable in co-precipitation experiments performed under the conditions set forth above.
However, in accordance with the present invention it was unexpectedly found that chaperones belonging to the family of heat shock protein (Hsp) 70 chaperones may also form complexes with (poly)peptides which lead to the ready detection of said chaperones in co-precipitations experiments performed under the conditions set forth above. These complexes are referred to as "stable complexes" in accordance with the present invention.
Moreover, it could be demonstrated that the formation of a stable complex of the chaperone with a (poly)peptide correlates not only with an increased stability of the (poly)peptide but, surpsisingly, also with the increased stability of a fusion protein comprising a first (poly)peptide which as such is unstable in a cell having a half-time of less than two hours and a second (poly)peptide forming said stable complex with said chaperone. Thus, the polynucleotide of the present invention may, e.g., be advantageously used to express said fusion protein in a cell to high levels which may exceed 0.1 µg/10⁶ cells.
In addition, as will be discussed in more detail below, the polynucleotide of the present invention can be advantageously used to present said fusion protein to the immune system of a subject, thereby triggering a humoral and/or cellular immune response against said first and second (poly)peptide. This application of the polynucleotide of the present invention is of particular advantage in cases where said first (poly)peptide does not induce an immune response when expressed by conventional methods already known in the art.

Since fusion of a first (poly)peptide to a second (poly)peptide which stably interacts with a chaperone also confers increased stability to said first (poly)peptide, the polynucleotide of the present invention is especially useful for the expression of (poly)peptides which are otherwise unstable and which, therefore, are only difficult to express per se or which can not be expressed at all.

In a most preferred embodiment of the polynucleotide of the present invention, said first (poly)peptide is or comprises an epitope and/or a functional and/or structural domain of a protein, and/or a mutated or truncated variant of a protein.

In a most preferred embodiment of the polynucleotide of the present invention, said chaperone is hsp73.

In a more preferred embodiment of the polynucleotide of the present invention, the function and/or structure of the N-terminal J domain of said viral T antigen is maintained.

J domains, originally discovered in DnaJ chaperones and mediating the interaction of these chaperones with DnaK chaperones, have also been identified in viral T antigens where they appear to have a similar function (for a recent review see, e.g., Brodsky, J.L., and J.M. Pipas, J.Virol. 72 (1998):5329).

In an additional more preferred embodiment of the polynucleotide of the present invention, said viral T antigen is a viral large T antigen.
Although large T antigen is the preferred viral T antigen, other viral T antigens like the middle, small, and tiny T antigen may also be used in accordance with the present invention.

In general, T antigens of members of the subfamily Polyomavirinae like, e.g., the murine polyomavirus (PyV) may be used in accordance with the present invention. However, in yet another more preferred embodiment of the polynucleotide of the present invention, said viral T antigen is SV40 T antigen.

In a most preferred embodiment of the polynucleotide of the present invention, said viral large T antigen is SV40 large T antigen.

In another most preferred embodiment of the polynucleotide of the present invention, the about 300 C-terminal amino acids of said SV40 large T antigen are deleted.
It was unexpectedly found in accordance with the present invention that mutant SV40 large T antigens carrying a deletion of the about 300 C-terminal amino acids but not full-length wt SV40 large T antigen show the above described surprising properties. SV40 large T antigen mutants carrying essentially a deletion as described above but e.g. retain the last about 10 or even less amino acids of the full-length wt SV40 large T antigen C-terminus fall under the definition of variants of the mutants of the present invention and, thus, are also comprised by the present invention.

Thus, in yet another most preferred embodiment of the polynucleotide of the present invention, said SV40 large T antigen contains amino acids 1 to 272. In still another most preferred embodiment of the polynucleotide of the present invention, the internal deletion comprises at least part of the nuclear localisation signal.
The term "nuclear localisation signal", as used in accordance with the present invention denotes an amino acid motif within a (poly)peptide which directs the transport of said (poly)peptide into the nucleus of a cell (see, e.g., Stryer L. (ed.), Biochemistry, 1995, W.H. Freeman and Company, New York). Different kinds of nuclear localisation signals (NLS) are known to the person skilled in the art and may, e.g., contain five consecutive positively charged amino acid residues like the SV40 large T antigen NLS: Lys-Lys-Lys-Arg-Lys.
It was surprisingly found that, in addition to C-terminal deletions, internal deletions which comprise at least part of the nuclear localisation signal of the SV40 large T antigen have, according to the present invention, similar beneficial effects. Without wanting to be bound to a specific scientific theory, it thus appears that conformational changes are induced by the above described deletions alone or in combination which render the corresponding mutants capable of forming stable complexes with chaperones belonging to the family of heat shock protein (Hsp) 70 chaperones.

In a further most preferred embodiment of the polynucleotide of the present invention, amino acids 110 to 152 are deleted.

In another preferred embodiment of the present invention the polynucleotide further encodes a tag.
As used in accordance with the present invention, the term "tag" denotes an amino acid sequence which can be introduced into the sequence of a (poly)peptide and used directly or indirectly, e.g., to label said (poly)peptide. Other examples of tags like, e.g., His-tags are well known to the person skilled in the art and can be used to facilitate isolation/purification of the fusion protein or the first (poly)peptide of the present invention.

In still another preferred embodiment of the polynucleotide of the present invention, said first and second (poly)peptide are linked via a protease cleavage site.

The incorporation of a protease cleavage site between said first and second (poly)peptide is especially useful if said first (poly)peptide is the desired end product. Thus, after synthesis of the fusion protein said second (poly)peptide may be easily separated from said first (poly)peptide by cleaving said protease cleavage site with an appropriate protease. For example, an appropriate protease cleavage site may comprise the amino acid sequence Lys-Asp-Asp-Asp-Asp-Lys which is specifically recognized by bovine enterokinase.

The polynucleotide of the present invention encoding the above described fusion protein may be, e.g., DNA, cDNA, RNA or synthetically produced DNA or RNA or a recombinantly produced chimeric nucleic acid molecule.

Furthermore, the present invention relates to a vector, particularly to a plasmid, a cosmid, a virus or a bacteriophage used conventionally in genetic engineering that comprise the polynucleotide of the invention.

In a preferred embodiment of the vector of the present invention, the polynucleotide is operatively linked to an expression control sequence.
Such an expression vector and/or gene transfer or targeting vector, derived, e.g., from a virus such as a retrovirus, vaccinia virus, adeno-associated virus, herpes virus, or bovine papilloma virus, may be used for delivery of the polynucleotides or vector of the invention into a targeted cell population. Methods which are well known to those skilled in the art can be used to construct recombinant viral vectors; see, for example, the techniques described in Sambrook et al., Molecular Cloning A Laboratory Manual, Cold Spring Harbor Laboratory (1989) N.Y. and Ausubel et al., Current Protocols in Molecular Biology, Green Publishing Associates and Wiley Interscience, N.Y. (1989). Alternatively, the polynucleotides and vectors of the invention can be reconstituted into liposomes for delivery to target cells. The vectors containing the polynucleotides of the invention can be transferred into the host cell by well-known methods, which vary depending on the type of cellular host. For example, calcium chloride transfection is commonly utilized for prokaryotic cells, whereas, e.g., calcium phosphate or DEAE-Dextran mediated transfection or electroporation may be used for other cellular hosts; see Sambrook, supra.
Such vectors may comprise further genes such as marker genes which allow for the selection of said vector in a suitable host cell and under suitable conditions. Preferably, the polynucleotide of the invention is operatively linked to expression control sequences allowing expression in prokaryotic or eukaryotic cells. Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory sequences ensuring initiation of transcription and, optionally, a poly-A signal ensuring termination of transcription and stabilization of the transcript, and/or an intron further enhancing expression of said polynucleotide. Additional regulatory elements may include transcriptional as well as translational enhancers, and/or naturally-associated or heterologous promoter regions. Possible regulatory elements permitting expression in prokaryotic host cells comprise, e.g., the PL, lac, trp or tac promoter in E. coli, and examples for regulatory elements permitting expression in eukaryotic host cells are the AOX1 or GAL1 promoter in yeast or the CMV-, SV40- , RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the polynucleotide of the invention and are well known in the art The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an C- or N-terminal identification peptide imparting desired characteristics, e.g., stabilization or, as mentioned above, simplified purification of expressed recombinant product. In this context, suitable expression vectors are known in the art such as Okayama-Berg cDNA expression vector pcDV1 (Pharmacia), pCDM8, pRc/CMV, pcDNA1, pcDNA3 (In-vitrogene), pSPORT1 (GIBCO BRL) or pCl (Promega).
Preferably, the expression control sequences will be eukaryotic promoter systems in vectors capable of transforming or transfecting eukaryotic host cells, but control sequences for prokaryotic hosts may also be used.
As mentioned above, the vector of the present invention may also be a gene transfer or targeting vector. Gene therapy, which is based on introducing therapeutic genes into cells by ex-vivo or in-vivo techniques is one of the most important applications of gene transfer. Suitable vectors and methods for in-vitro or in-vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Isner, Lancet 348 (1996), 370-374; Muhlhauser, Circ. Res. 77 (1995), 1077-1086; Wang, Nature Medicine 2 (1996), 714-716; WO94/29469; WO 97/00957 or Schaper, Current Opinion in Biotechnology 7 (1996), 635-640, and references cited therein. The polynucleotides and vectors of the invention may be designed for direct introduction or for introduction via liposomes, or viral vectors (e.g. adenoviral, retroviral) into the cell. Preferably, said cell is a germ line cell, embryonic cell, or egg cell or derived therefrom, most preferably said cell is a stem cell.

In another embodiment the present invention relates to a host cell comprising the polynucleotide or the vector of the present invention.

In a preferred embodiment the host cell is a prokaryotic or eukaryotic cell.
The polynucleotide or vector of the invention which is present in the host cell may either be integrated into the genome of the host cell or it may be maintained extrachromosomally.
The host cell can be any prokaryotic or eukaryotic cell, such as a bacterial, insect, fungal, plant, animal or human cell. Preferred fungal cells are, for example, yeast cells of the genus Hansenuia or Saccharomyces, in particular those of the species S. cerevisiae. The term "prokaryotic" is meant to include all bacteria which can be transformed or transfected with the polynucleotide or vector of the present invention. Prokaryotic hosts may include gram negative as well as gram positive bacteria such as, for example, E. coli, S. typhimurium, Serratia marcescens and Bacillus subtilis. The term "eukaryotic" is meant to include yeast, higher plant, insect (e.g. Schneider cells), and preferably mammalian cells. Examples of eukaryotic cells to be employed in accordance with the present invention are TC7 cells (African green monkey kidney cells), L929 cells (murine fibroblasts), Hela cells (human epithelial cells) or LMH cells (chicken hepatoma cells). However, other cells derived from other cell lines, tissues, and/or species may also be advantageously used in accordance with the present invention.

The present invention, furthermore, relates to a method for the production of the fusion protein to be employed in accordance with the polynucleotide of the present invention, said method comprising culturing the host cell of the present invention under conditions that allow the synthesis of said fusion protein, and recovering said fusion protein from the culture.
As will be readily appreciated by the person skilled in the art, depending on the expression system used, the synthesized fusion protein will be secreted into the culture medium or will accumulate in the cells. Thus, the term "culture" as used in accordance with the present invention comprises the culture medium and/or the cells used to synthesize the (poly)peptide of the invention. Suitable protocols for the recovery of (poly)peptides from the culture medium or from cells, including the disruption or lysis of the cells, are well known to the person skilled in the art (see, e.g., Sambrook et al., Molecular Cloning A Laboratory Manual, Cold Spring Harbor Laboratory (1989) N.Y. and Ausubel et al., Current Protocols in Molecular Biology, Green Publishing Associates and Wiley Interscience, N.Y. (1989)).

In a preferred embodiment, the method of the present invention further comprises the step of separating said fusion protein from complexed chaperones.
The person skilled in the art is well aware of methods suitable for the disruption of (poly)peptide/chaperone complexes and the isolation of (poly)peptides from complexing chaperones. Such methods include, e.g., adenosine triphosphate (ATP)-affinity chromatography which leads to the dissociation of chaperones from (poly)peptides.

The present invention also relates to a fusion protein encoded by the polynucleotide or the vector of the present invention, or obtainable or obtained by the method of the present invention.

In an additional embodiment the present invention relates to a method for the production of a first (poly)peptide as defined hereinabove, said method comprising culturing the host cell of the present invention under conditions that allow the synthesis of the fusion protein as described hereinabove, recovering said fusion protein from the culture, and separating said second (poly)peptide from said fusion protein by proteolytic cleavage.

In addition, the present invention relates to a method for the production of a complex comprising the fusion protein of the present invention and a chaperone belonging to the family of heat shock protein (Hsp) 70 chaperones to be employed in accordance with the polynucleotide of the present invention, said method comprising culturing the host cell of the present invention under conditions that allow complex formation of said fusion protein with said chaperone, and recovering said complex from the culture.
Methods for the isolation of (poly)peptide/chaperone complexes are well known in the art and include, e.g., adenosine diphosphate (ADP)-affinity chromatography (see, e.g., Peng, P., A. Menoret, and P. K. Srivastava, J. Immunol. Methods 204 (1997):13).

The present invention additionally relates to a method for the production of an antibody directed against a first (poly)peptide as defined hereinabove, said method comprising administering in an amount sufficient to induce a humoral immune response the polynucleotide, the vector and/or the fusion protein of the present invention, and/or the first (poly)peptide and/or the complex obtainable or obtained by the method of the present invention to a non-human subject.
In accordance with the present invention, it was surprisingly found that genetic immunization with the nucleic acid molecules of the present invention not only leads to a cellular but also to a humoral immune response. The immunogenicity of endogenous peptide/protein antigens for T and B cells was strikingly altered by stable binding to a chaperone belonging to the family of heat shock protein (Hsp) 70 chaperones. Alternative intracellular processing pathways control the activation of MHC-ll-restricted CD4⁺ and MHC-I-restricted CD8⁺ T cell responses to protein antigens. In the exogenous pathway, endocytosed antigens are partially degraded in a specialized endosomal compartment of APCs to 12 to 15 residue peptides by acid proteolysis. Soluble protein antigens processed in this pathway elicit CD4⁺ T cell and antibody responses. In contrast, CD8⁺ CTL responses are stimulated by antigens processed in the alternate endogenous pathway in which peptides derived from cytosolic proteins are transported into the ER lumen by a peptide transporter complex where they bind to nascent MHC class I heavy chain/β₂m microglobulin dimers. This generates trimeric, transport-competent MHC class I complexes that move rapidly by the default secretory route to the cell surface. Antigens from an exogenous or an endogenous source thus seem to be processed in alternative, mutually exclusive pathways for MHC-restricted presentation of antigenic peptides to T cells. As could be shown in accordance with the present invention, this rule is bypassed when some endogenous proteins are mutated or truncated and thereby gain a stable chaperone-binding phenotype. In this way, mutant oncogenes, tumor-associated antigens, self antigens or non-structural viral antigens may elicit antibody responses in tumor-bearing or infected hosts. It has been puzzling that some dominant autoantibody responses in systemic autoimmune diseases are directed against cytosolic or nuclear autoantigens. The chaperone-facilitated presentation of endogenous antigens to B cells also offers an attractive interpretation of some of the described phenomena.
As could be shown in accordance with the present invention in genetic vaccination experiments (see Examples 8 and 9), nucleic acid molecules encoding a second (poly)peptide or a fusion protein comprising a second (poly)peptide as defined hereinabove effectively elicited a humoral immune response against said second (poly)peptide and/or the fusion partner, whereas nucleic acid molecules encoding (poly)peptides not capable of stably interacting with the chaperone did not. Thus, it is envisaged in accordance with the present invention that the polynucleotide of the present invention can be advantageously used for the production of antibodies directed against a (poly)peptide which, if expressed alone or as conventional fusion protein with another (poly)peptide, which does not stably interact with the chaperone, does not elicit a humoral immune response.

As regards the routes of Administration, dosages, etc., the person skilled in the art is well aware of the different possibilities and the factors which influence the choice (for a detailed discussion, see below).

In an additional embodiment the present invention relates to a kit comprising the polynucleotide, the vector, the host cell, and/or the fusion protein of the present invention, and/or the first (poly)peptide, the complex, and/or the antibody obtainable or obtained by the method of the present invention.

In a further embodiment the present invention relates to a diagnostic composition comprising the polynucleotide, the vector, and/or the fusion protein of the present invention, and/or the first (poly)peptide, the complex, and/or the antibody obtainable or obtained by the method of the present invention.
The diagnostic composition optionally comprises suitable means for detection. The (poly)peptides and antibodies described above are, for example, suited for use in immunoassays in which they can be utilized in liquid phase or bound to a solid phase carrier. Examples of immunoassays which can utilize said (poly)peptide are competitive and non-competitive immunoassays in either a direct or indirect format. Examples of such immunoassays are the radioimmunoassay (RIA), the sandwich (immunometric assay) and the Western blot assay. The (poly)peptides and antibodies can be bound to many different carriers and used to isolate cells specifically bound to said polypeptides. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention.
There are many different labels and methods of labeling known to those of ordinary skill in the art. Examples of the types of labels which can be used in the present invention include enzymes, radioisotopes, colloidal metals, fluorescent compounds, chemiluminescent compounds, and bioluminescent compounds.
Said diagnostic compositions may also be used for methods for detecting expression of a polynucleotide of the invention by detecting the presence of mRNA coding for the corresponding (poly)peptide, said method comprising obtaining mRNA from a cell and contacting the mRNA so obtained with a probe comprising a nucleic acid molecule of at least 15 nucleotides capable of specifically hybridizing with a polynucleotide of the invention under suitable hybridizing conditions (regarding "suitable hybridizing conditions", see, e.g., Sambrook et al., Molecular Cloning A Laboratory Manual, Cold Spring Harbor Laboratory (1989) N.Y., or Higgins and Hames (eds), Nucleic Acid Hybridization, A Practical Apprach, IRL Press Oxford (1985)), detecting the presence of mRNA hybridized to the probe, and thereby detecting the expression of the (poly)peptide by a cell.

The components of the kit or the diagnostic composition of the present invention may be packaged in containers such as vials, optionally in buffers and/or solutions. If appropriate, one or more of said components may be packaged in one and the same container. Additionally or alternatively, one or more of said components may be adsorbed to a solid support such as, e.g., a nitrocellulose filter or nylon membrane, or to the well of a microtitre-plate.

The present invention also relates to a method for the detection of the presence of an epitope comprised in a (poly)peptide as defined hereinabove, said method comprising contacting the fusion protein of the present invention or the first (poly)peptide obtainable or obtained by the method of the present invention with an antibody or a cytotoxic T-lymphocyte (CTL) under conditions that allow binding of said antibody or CTL to said epitope, and detecting whether the antibody or CTL has bound to said epitope.

Thus, the method of the present invention may be used to screen selected domains, fragments or epitopes of complex endogenous antigens for immunogenicity for T and B cells. This extends the use of DNA vaccination strategies based on 'minigene' constructs. When cDNA expression libraries are used in DNA immunization to search for immunogenic products of a pathogen, pools of cDNA fragments fused C-terminally to a second (poly)peptide as described above will be stably expressed to levels required for efficient priming of immune responses, and will thus substantially increase the repertoire of Immunogenic sites that are potentially useful in vaccine designs.
Methods for the detection and mapping of CTL epitopes are well known in the art and include, e.g., the cytotoxicity or ⁵¹Cr release assay (for a discussion of the theoretical principle of this assay see, e.g., Roitt, I., at al. (eds.), Immunology, Gower Medical Publishing, Ltd., London, England (1985)).

In a preferred embodiment of the method of the present invention, said antibody or CTL is derived from an individual infected with a pathogen.

In another preferred embodiment of the method of the present invention, the first (poly)peptide of said fusion protein or said first (poly)peptide obtainable or obtained by the method of the present invention is derived from a pathogen.

The present invention also relates to a pharmaceutical composition comprising the polynucleotide, the vector, and/or the fusion protein of the present invention, and/or the first (poly)peptide, the complex, and/or the antibody obtainable or obtained by the method of the present invention, and, optionally, a pharmaceutically acceptable carrier and/or diluent
Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. The dosage regimen will be determined by the attending physician and clinical factors. As is well known in the medical arts, dosages for any one patient depends upon many factors, including the patients size, body surface area, age, the particular compound to be administered, sex, time and route of administration, general health, and other drugs being administered concurrently. A typical dose can be, for example, in the range of 0.001 to 1000 µg (or of nucleic acid for expression or for inhibition of expression in this range); however, doses below or above this exemplary range are envisioned, especially considering the aforementioned factors. Generally, the regimen as a regular administration of the pharmaceutical composition should be in the range of 1µg to 10 mg units per day. If the regimen is a continuous infusion, it should also be in the range of 1 µg to 10 mg units per kilogram of body weight per minute, respectively. Progress can be monitored by periodic assessment Dosages will vary but a preferred dosage for intravenous administration of DNA is from approximately 10⁶ to 10¹² copies of the DNA molecule. The compositions of the invention may be administered locally or systemically. Administration will generally be parenterally, e.g., intravenously; DNA may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the invention may comprise further agents such as interleukins or interferons depending on the intended use of the pharmaceutical composition.

In a preferred embodiment of the present invention the pharmaceutical composition is a vaccine.

In a more preferred embodiment of the present invention said vaccine induces a humoral and/or cellular immune response.

Additionally, the present invention relates to the use of the polynucleotide or the vector of the present invention for the production of an antibody directed against a first (poly)peptide as defined hereinabove.

Finally, the present invention relates to the use of a (poly)peptide comprising an epitope detected by the method of the present invention or a complex produced by the method of the present invention for the production of an antibody.

Abbreviations used in this application: CTL, cytotoxic T lymphocyte; MHC, major histocompatibility complex, MHC-I, MHC class I molecule; MHC-II, MHC class II molecules; Hsp, heat shock protein; SV40 simian virus 40; T-Ag, large tumor antigen of SV40; c, cytolasmic (truncated); wt, wild-type; T272. a truncated T-Ag variant comprising the N-terminal 272 residue; TAP, transporter associated with antigen processing; ER, endoplasmic reticulum.

The figures show:

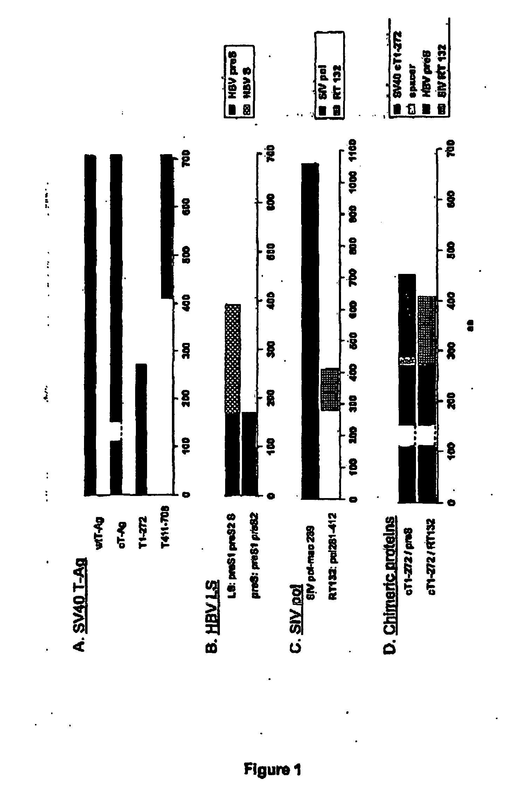

**Figure 1****: Recombinant antigens used for nucleic acid immunization and transfection.** The antigens used in the present study were derived from: **(A)** the simian virus 40 large tumor antigen (SV40 T-Ag), **(B)** the hepatitis B virus large surface protein (HBV-LS) and **(C)** the simian immunodeficiency virus (mac239) polymerase (SIV-pol). **(D)** In addition, chimeric proteins containing an N-terminal cytoplasmic T-Ag (cT-Ag) fragment (from amino acid position 1 to 272) and either the N-terminal preS fragment, or the internal RT132 fragment are shown. As indicated, this cT-Ag variant contained a 43 residue deletion (aa 110-152) of the nuclear localisation signal leading to an overall lenght of said cT-Ag fragment of 229 amino acids. Generation of these recombinant constructs and the exact amino acid sequences are described in Example 1.

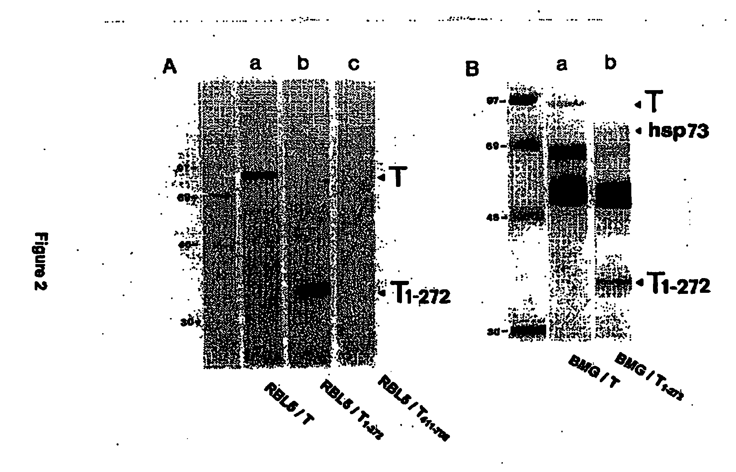

**Figure 2****: T-Ag expressing transfectants.** (A) RBL5 cells were transfected with BMG/T (lane a; RBL5/T), BMG/T272 (lane b; RBL5/T1-272), or BMG/T411-708 (lane c; RBL5/T411-708). G418-resistant, stable transfectants (5x10⁸ cells) were lysed in SDS-containing buffer and samples (corresponding to 10 µg protein) were processed for SDS-PAGE and Western blotting using a rabbit anti-T-Ag antiserum. Antibodies against the C-terminus of T-Ag also failed to detect expression of the T411-708 fragment (data not shown). Similar data were obtained using cells transiently transfected with either BMG-, or pCl-based expression constructs (data not shown). (B) LMH cells were transiently transfected with the T-Ag-encoding BMG/T vector (lane a) or the T1-272-encoding BMG/T272 vector (lane b). 2x10⁶ cells were extracted 48 h post-transfection with lysis buffer and immunoprecipitated for T-Ag using mAb PAb108 and protein A-Sepharose. Immune complexes were processed for SDS-PAGE followed by Coomassie Blue staining of the gel. The positions of hsp73, T-Ag (T) and T1-272 are indicated.

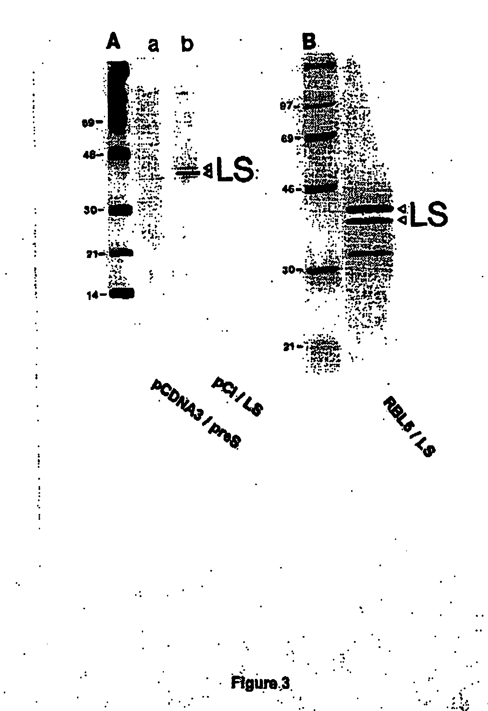

**Figure 3****: PreS-Ag expressing transfectants. (A)** LMH cells were transiently transfected with either the preS-encoding pcDNA3/preS plasmid (lane a), or the LS-encoding pCl/LS plasmid (lane b). 2×10⁶ cells were lysed 48 h after the transfection in SDS-containing buffer. Samples corresponding to 10 µg protein were processed for SDS-PAGE and Western blotting using a rabbit anti-preS1 antiserum. **(B) RBL5** cells were stably transfected with BMG/LS. Stable RBL5/LS transfectants (4×10⁶ cells) were labeled for 1 h with ³⁵S-methionine, extracted with lysis buffer and immunoprecipitated for LS-antigen using an rabbit anti-S antiserum and protein A-Sepharose. Immune complexes were processed for SDS-PAGE and fluorography. The positions of glycosylated and non-glycosylated LS antigen is indicated.

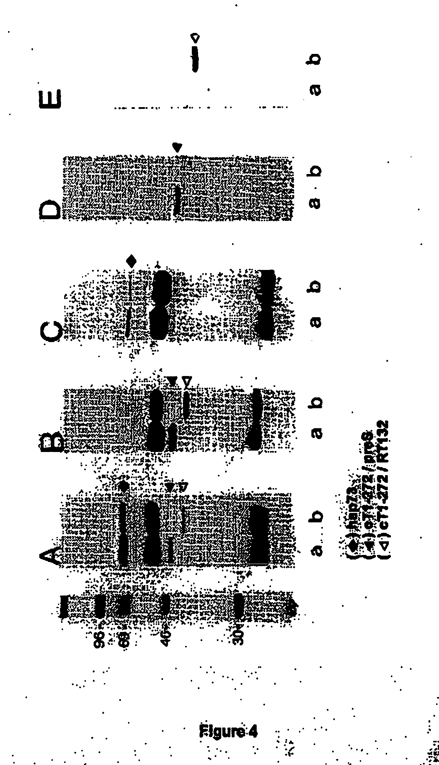

**Figure 4****: Expression of chimeric proteins In transfectants.** RBL5 cells were transfected with the BMG/cT1-272preS plasmid (lanes a) or the BMG/cT1-272RT132 plasmid (lanes b). Stable, G418-resistant transfectants were extracted with lysis buffer and immunoprecipitated for T-Ag using mAb PAb108 and protein A-Sepharose. Immune complexes were processed for SDS-PAGE followed by Coomassie Blue staining of the gel (A), or by Western blotting **(B-E).** For detection of T-Ag sequences in the fusion proteins, we used the anti-T-Ag mAb 108 and rabbit anti-mouse immunoglobulin antiserum **(B);** for detection of preS sequences, we used a rabbit anti-preS1 antiserum (D); for detection of RT sequences, we used a rabbit anti-RT antiserum (E). Complex formation of the chimeric proteins with hsp73 was confirmed with the anti-hsp70 mAb 3A3 and rabbit anti-mouse immunoglobulin antiserum **(C).** The positions of hsp73, cT1-272/preS and cT1-272 /RT132 are indicated.

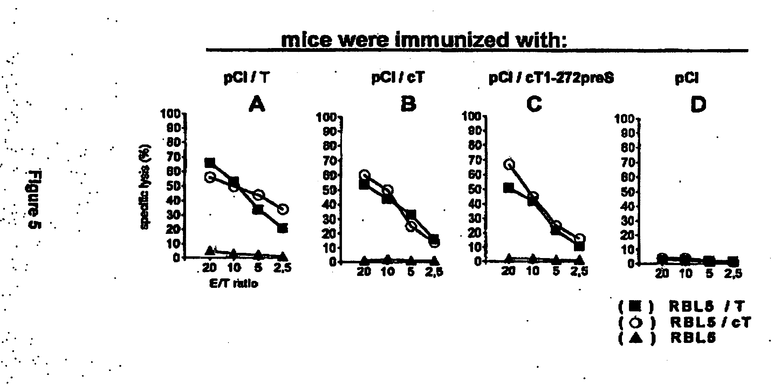

**Figure 5****: CTL response of C57BL/6 (H-2^{b}) mice induced by vaccination with plasmid DNA encoding T-Ag**. Mice were injected intramuscularly with either 100 µg of wtT-Ag-encoding pCI/T **(A),** cT-Ag-encoding pCl/cT **(B),** cT1-272preS-encoding pCl-cT1-272preS **(C),** or the pCl vector control without insert **(D).** Splenic CTL obtained from mice 3 weeks post-vaccination were restimulated *in vitro* for 5 d with inactivated wtT-Ag-expressing EL4/T cells, and their specific cytolytic reactivity was measured in a 4 h ⁵¹Cr-release assay against RBL5 targets expressing T-Ag (RBL5/T), cT-Ag (RBL5/cT) and BMGneo-transfected control RBL5 targets (RBL5). Plotted lysis values at the Indicated effector/target ratios represent means of triplicates.

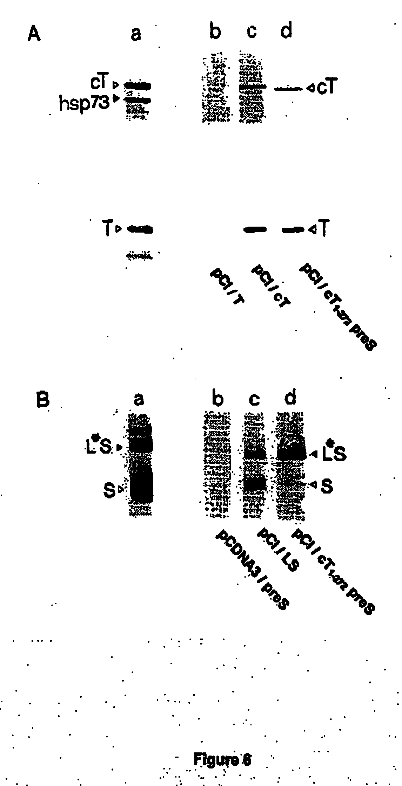

**Figure 6****: Serum antibody response of C57BL/6 (H-2^{b}) mice elicited by vaccination with plasmid DNA. (A)** *T-Ag* response: Mice were injected intramuscularly with either 100 pg of wtT-Ag-encoding pCl/T (lanes b), cT-Ag-encoding pCl/cT (lanes c), or cT1-272preS-encoding pCl/cT1-272preS (lanes d). Serum antibody titers were determined in Western blot analyses using immunoprecipitated cT-Ag- hsp73 (upper panel) or wtT-Ag (lower panel) samples (lanes a). **(B)** *preS-Ag response:* Mice were injected intramuscularly with either 100 µg of preS-encoding pcDNA3/preS (lane b), LS-encoding pCl/LS (lane c), or cT1-272preS-encoding pCl/cT1-272preS (lane d). Serum antibody titers were determined by Western blot analyses using SDS- and mercaptoethanol- denatured L*S particles (lanes a). Sera were obtained from mice at 10 weeks post-vaccination and diluted 1:100 for Western analyses. The data shown were obtained from individual, representative mice. The positions of hsp73, cT-Ag, T-Ag, preS-containing L*S-Ag (L*S) and of the small S-Ag (S) are indicated.

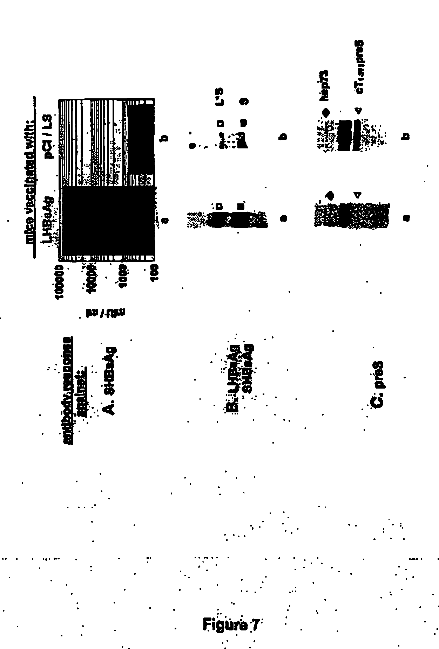

**Figure 7****: Anti-S and anti-preS serum antibody responses of mice vaccinated** with **L-encoding plasmid DNA or L/M/S-containing particles.** Mice were immunized with either 50 µg CHO-derived L/M/S particles emulsified in alum (lanes a), or 100 µg L-encoding pCI/LS plasmid DNA (lanes b). Sera were obtained from mice at 12 weeks post-vaccination. Anti-S antibody tiers (mlU/ml) from individual mice determined in the IMxAUSAB test are shown (A). Anti-S and anti-L antibody induction was furthermore determined from the same individual mouse in Western blot analyses using SDS-/mercaptoethanol-denatured L*S particles **(B).** Anti-preS antibody induction in the same individual mouse was determined in Western blot analyses using hsp73-associated cT1-272preS protein as the test antigen **(C).** All sera were diluted 1:100 in the shown Western analyses. The positions of hsp73, cT1-272preS, preS-containing L*S-Ag (L*S) and S are indicated. Data from two representative mice out of a group of 14 analyzed mice are shown.

The examples illustrate the invention.

### Example 1: Mice, cell lines, and vectors used for transfection of cell lines.

Mice: C57BL/6J (B6) mice (H-2^{b}) or BALB/c mice (H-2^{d}) were bred and kept under standard-pathogen-free conditions in the animal colonies of Ulm University (Ulm, Germany). Breeding pairs of these mice were obtained from Bomholtgard (Ry, Denmark). Female mice were used at 10-16 weeks of age.
Cell lines: The H-2^{b} thymoma line EL4 (TIB-39) was obtained from the American Tissue Culture Collection (ATCC, Rockville, MD). The H-2^{b} (C57BL/6-derived) T lymphoma cell line RBL5 was generously provided by Dr. H.-U. Weltzien (Freiburg, Germany), LMH chicken hepatoma cells were from Dr. H.-J. Schlicht (Ulm, Germany).
Vectors used for transfection of cell lines: The BMGneo vector system was used for the generation of stable antigen-expressing transfectants in which genes are expressed under methallothionin promoter control (Karasuyama, H. and F. Melchers, Eur.J.Immunol. 18 (1988):97). The wtT-Ag of SV40, the mutant cytoplasmic cT-Ag variant (with a deletion of SV40 nucleotide position 4490-4392, *i.e.* amino acid position 110-152 of the T-Ag), or the N-terminal T1-272 fragment (T1-272) were cloned into the BMGneo vector as described (Schirmbeck, R., J. Zerrahn, A. Kuhröber, E. Kury, W. Deppert, and J. Reimann, Eur.J.Immunol. 22 (1992):759; Schirmbeck, R., W. Deppert, E. Kury, and J. Reimann, Cell.Immunol. 149 (1993):444; Schirmbeck, R., J. Zerrahn, A. Kuhröber, W. Deppert, and J. Reimann, Eur.J.Immunol. 23 (1993):1528). The BMG/T411-708 construct was generated from the T-Ag-encoding plasmid pEARLY generously provided by Drs. W. Deppert and V. von Hoyningen (Hamburg, Germany) (von Hoyningen-Huene, V., M. Kurth, and W. Deppert, Virology 190 (1992):155). The T411-708-encoding Nsil/BamHI fragment of pEARLY was cloned into the Pstl/BamHI site of pBluescript. The resulting plasmid pBlueT411-708 was cut with Xhol and BamHI and cloned Into Xhol/BamHI site of the BMGneo vector generating the plasmid BMG/T411-708. The BMG/LS construct was generated from plasmid pTKTHBV2 (a generous gift of Dr. M. Meyer, Munich, Germany) and the HBsAg-encoding BMG/S vector (Schirmbeck, R., K. Melber, A. Kuhröber, Z.A. Janowicz, and J. Reimann, J.Immunol. 152 (1994):1110; Schirmbeck, R., K. Melber, T. Mertens, and J. Reimann, J. Virol. 68 (1994):1418). The preS1/preS2-encoding BgIII/Xbal-fragment of HBV, subtype ayw, was cloned into the BgIII/Xbal site of pBluescript. The resulting plasmid preS/Blue was cut with Xhol and cloned into the Xhol site of BMG/S yielding the plasmid BMG/LS. The vector BMG/cT1-272RT132 was generated from a cT-Ag encoding plasmid cyBlue and a 400 bp EooRI-fragment encoding the aa sequence 281-412 of SIVmac239 polymerase (a generous gift of Drs. H. Petry, Göttingen and K. Melber, Düsseldorf). The EcoRI-fragment was cloned into EcoRl site of pBluescript generating the plasmid RT132Blue. This plasmid was cut with HindIII/BamHI to obtain the RT132-encoding 400 bp fragment that was coned into the HindIII/BcIII site of cyBlue. The resulting plasmid was linearized with HindIII and fused with a cT1-272- encoding HindIII-fragment form cyBlue to generate the plasmid cT1-272RT132. The cT1-272RT132 plasmid was cut with XhoI/BamHI and cloned into XhoI/BamHI cut BMGneo yielding the plasmid BMG/cT1-272RT132, which encodes the following amino acid sequence (see Fig. 1D): SV40: cT1-272 ; spacer. DIEF ; SIVpol281-412 sequence MLIDFRELNRVTQDFTEVOLGIPHPAGLAKRKRITVLDIGDAYFSIPLDEEFRQYTAFT LPSVNNAEPGKRYIYKVLPQGWKGSPAIFQYTMRHVLEPFRKANPDVTLVQYMDDI LIASDRTDLEHDRVVL; stop: DPGGS. The spacer and stop sequences were not related to T-Ag or pol. The HBVpreS- encoding sequence was generated from the plasmid preS/Blue (see above) by PCR using primers 5' TCGA**ATG**GGGCAG**AAT**CTTTCCAC 3' and 3' CCCTGGGACGCGACTRG**ATT**TCGA 5'. The corresponding start and stop signals of the preS antigen are indicated. The PCR product was cloned into EcoRV-cut pBluescript yielding the plasmid preSstop/Blue. The plasmid preSstop/Blue was digested with Sall and the 5' ends were filled with Klenow polymerase followed by Kpnl digestion. A cT1-272 encoding Kpnl / Smal fragment was cloned into the Kpnl-Sall/blunt preSstop/Blue vector generating the plasmid cT1-272preSBlue. The plasmid cT1-272preS/Blue was cut with Sall and the 5' ends were filled with Klenow polymerase, digested with Smal and cloned into the Xho-cut and blunted BMGneo vector yielding plasmid BMG/cT1-272preS. This encodes the following amino acid sequence (see Fig. 1D): SV40: cT1-272 ; spacer: DIEFLQPSTVSISLIR; the HBV preS: MGQNLSTSNPLGFFPDHQLDPAFRANTANPDWDFNPNKDTWPDAANKVGA GAFGLGFTPPHGGLLGWSPOAQGILQTLPANPPPASTNROSGROPTPLSPPLRNT HPQAMQWNSTTFHQTLQDPRVRGLYFPAGGSSSGTVNPVLTTASPLSSIFSRSDP ALN. The spacer sequence was not related to T-Ag or preS.

### Example 2: Vectors used for nucleic acid Immunization, vaccination of mice, and characterization of antigen expression in transfected cells.

Vectors used for nucleic acid immunization: Vectors used for nucleic and immunization were based on the pCl expression vector (cat.no. E1731, Promega, Heidelberg, FRG) or the pcDNA3 vector (invitrogen). All plasmids expressed the antigen under the human CMV immediate early promoter control. pCI/T vector was generated from a T-Ag-encoding Sall/BamHI fragment of BMG/T vector which was cloned into XhoI/BamHI I digested pCI vector (Schirmbeck, R., W. Deppert, E. Kury, and J. Reimann, Cell.Immunol. 149 (1993):444; Schirmbeck, R., J. Zerrahn, A. Kuhröber, W. Deppert, and J. Reimann, Eur.J.Immunol. 23 (1993):1528). pCl/cT vector was generated from a cT-Ag-encoding SalI/BamHI fragment of BMG/cT vector which was cloned into XhoI/BamHI I digested pCl vector. The preS1/preS2-encoding Kpnl/XbaI-fragment of plasmid preSstop/Blue was cloned into KpnI/XbaI-cut pCDNA vector yielding the plasmid pCDNA3/preS. The preS1/preS2-encoding XhoI-fragment of plasmid preS/Blue was cloned in frame into Xhol-cut small S-Ag- encoding pCI/S vector, yielding the plasmid pCI/LS. pCl/cT1-272preS was generated from the plasmid cT1-272preS/Blue. Plasmid cT1-272preS/Blue was cut with Sall and Notl and cloned into Sal/tNotl digested pCI vector. The cT1-272RT132 plasmid was cut with XhoI/BamHI and cloned into XhoI/BamHI cut pCI yielding the plasmid pCI/cT1-272RT132.
Vaccination of mice: Plasmid DNA used for immunization was purified by anion exchange chromatography with the Qiagen maxi prep kit-(Qiagen, Hilden, Germany). We injected 50 pi of a 1 µg/µl plasmid DNA-in PBS solution into each non-pretreated tibialis anterior muscle (Böhm, W., A. Kuhröber, T. Paier, T. Mertens, J. Reimann, and R. Schirmbeck, J.Immunol.Methods 193 (1996):29). All mice received only one injection.
Characterization of antigen expression in transfected cells: Cells were transfected with plasmids using either the Ca₂PO₄ method, or DOTAP liposomes. Stable transfectant cell lines were established as described (Schirmbeck, R., J. Zerrahn, A. Kuhröber, E. Kury, W. Deppert, and J. Reimann, Eur.J.Immunol. 22 (1992):759; Schirmbeck, R., K. Melber, A. Kuhröber, Z.A. Janowicz, and J. Reimann, J.Immunol. 152 (1994):1110). Where indicated, cells (2-5 x 10⁶ cells) were transfected transiently for 48-60 hours. For immunoprecipitation analyses transfected cells were extracted with lysis buffer [120mM NaCl, 1% aprotinin (Trasylol, Bayer, Leverkusen, Germany), leupeptin, 0.5% NP40 and 50mM Tris-hydrochiohde (pH8.0)] for 30 min at 4°C. Extracts were cleared by centrifugation and immunoprecipitated for T-Ag using the mAb 108 directed against the extreme N-terminus of the T-Ag (Gumey, E.G., S. Tamowsky, and W. Deppert, J.Virol. 57 (1986):1168) or for LHBsAg using a polyclonal rabbit anti-S antiserum (Behringwerke, Marburg, Germany) and protein A-Sepharose. Immune complexes bound to protein A-Sepharose were purified with wash buffer [300mM LiCl, 1% NP40 and 100mM Tris-hydrochloride (pH 8.5)], followed by two washes with PBS and 0.1 x PBS. immune complexes were recovered from protein A-Sepharose with elution buffer [1.5% SDS, 5% mercaptoethanol and 7mM Tris-hydrochloride (pH6.8)] and processed for SDS-PAGE. Levels of immunoprecipitated proteins were analyzed either by Coomassie Blue staining of the gels and/or by Western blotting. Alternatively, transfected cells were lysed directly with an SDS-containing buffer [1.0% SDS, 5% mercaptoethanol and 50mM Tris-hydrochloride (pH7.0)]. Cell lysates were passed through a 21 gauge needle and cleared by centrifugation. Cleared lysates were boiled, and 5% Bromphenolblue in glycerin were added. Samples corresponding to 10 µg of protein were analyzed by SDS-PAGE and Western blotting.

### Example 3: Western blotting, and determination of serum antibody levels.

Western blotting: After SDS-PAGE, gels were incubated for 10 min in equilibration buffer [0.1% SDS, 20mM Tris-acetate (pH8.3)]. Proteins were electroblotted with a Transblot apparatus onto nitrocellulose paper at 60 V for 2 hours. The transfer buffer was 0.1% SDS, 20% isopropanol and 20 mM Tris-acetate (pH 8.3). Nitrocellulose sheets were incubated for 30 min with 50% isopropanol, for 15 min in TBS buffer [150mM NaCl, 5 mM NaNa, 1mM EDTA, 15 mM Tris-hydrochloride (pH 7.8)] and for 12 h in buffer G [TBS buffer + 0.1% gelatine + 100µg/ml immunoglobulin-free bovine serum albumin]. Nitrocellulose sheets were washed for 1 h with buffer GT [TBS buffer + 0.1% gelatine + 0.1% Tween 20]. The following polyclonal rabbit antisera were used: anti-T-Ag antiserum, anti- preS1 antiserum, anti-S antiserum, and anti-RT serum (generated against 200 µg SIVpol377-394 EPFRKANPDVTLVQYMDD peptide and 20 µg ovalbumin in 250 µl PBS, emulsified in 250 µl montanide™). Sheets were incubated for 4-6 h in GT buffer containing 1:500 diluted rabbit antisera. Alternatively, sheets were incubated for 2-3 h with murine mAbs (1:500) (i.e. anti- TAg mAb PAb 108, or anti- hsp 70 mAb 3A3 (cat. no, MA3-006; Dianova, Hamburg, Germany), or sera from immunized mice (1:50 - 1:500), washed and incubated for a further 2 h with 1:200 diluted rabbit anti-mouse antibodies in GT buffer (a generous gift of Dr. W. Deppert, Hamburg, Germany). Unbound antibodies were removed by washes in GT buffer. Thereafter, sheets were incubated for 2-12 h with 0.5-1 µCi of ³⁵S- labeled protein A (Amersham, Braunschweig, Germany), washed, dried, soaked in 20 % 2,5-diphenyloxazolol (PPO) in toluol and again dried. Radiolabeled immune complexes were detected by fluorography.
Determination of serum antibody levels: Sera were obtained from immunized mice 4-16 weeks post-vaccination. Antibodies against SV40 T-Ag, SV40 cT-Ag, hsp73, HBV preS or S-antigen were determined in Western blot anlyses. The T-Ag used for Western blotting was generated by immunoprecipitation of T-Ag - or cT-Ag-expressing cells. The preS-detecfing antigen used in Western blotting were yeast-derived L*S particles (Batch no. 25/8; a generous gift of Dr. P. Pala, SmithKline Beecham, Rixensart, Belgium). Western blotting was performed as described above.

### Example 4: In vitro restimulation of primed T-Ag specific CTL, and Cytotoxic assay.

In vitro restimulation of primed T-Ag specific CTL: Spleens were removed from immunized mice 3 weeks post-vaccination. Single cell suspensions were prepared in α-MEM tissue culture medium supplemented with 10 mM HEPES buffer, 5 x 10⁻⁵ M 2-ME, antibiotics and 10% v/v FCS (Gibco BRL, Eggenstein, Germany). A selected batch of Con A-stimulated rat spleen cell supernatant (2% v/v) was added to the culture medium. Three x 10⁷ responder cells were cocultured in a mixed lymphocyte tumor cell culture with 1 x 10⁸ irradiated, syngeneic T-Ag expressing EL4/T cells. Coculture was performed in 10 ml medium in upright 25 cm² tissue culture flasks in a humidified atmosphere/7% CO₂ at 37°C. After 5 days of culture, cells were harvested and used as effector cells in the cytotoxic assay.
Cytotoxic assay: Serial dilutions of effector cells were cultured with 2 x 10^{3 51}Cr-labeled targets in 200µl round-bottom wells. Specific cytolytic activity of cells was tested in a ⁵¹Cr-release assay against T-Ag expressing targets or non-transfected control targets. After a 4 h incubation at 37°C, 100 µl of supernatant were collected for γ-radiation counting. The percentage specie release was calculated as [(experimental release - spontaneous release) / (total release - spontaneous release)] x 100. Total counts were measured by resuspending target cells. Spontaneously released counts were always less than 15 % of the total counts. Data shown are the mean of triplicate cultures. The SEM of triplicate data was always less than 20 % of the mean.

### Example 5: Hsp73-facilitated expression of SV40 T-Ag variants with an Intact N-terminus.

Expression of the karyophilic simian virus 40 large tumor antigen (T-Ag) is readily detected in cells transfected with expression plasmids encoding wtT-Ag (Fig. 1A and 2A,B lane a). Mutant T-Ag variants with an intact N-terminus were as efficiently expressed as wtT-Ag in eukaryotic transfectants in which they accumulated to easily detectable steady state levels, This was shown using a cytoplasmic T-Ag (cT-Ag) variant containing a 43 residue deletion of the nuclear localisation signal, and an N-terminal 272 residue T-Ag fragment T1-272 (Fig. 1A). Whereas mutant cT-Ag or T1-272 protein were efficiently expressed in different cell lines (Fig. 2A,B, lanes b) (Schirmbeck, R. and J. Reimann, Eur.J.Immunol. 24 (1994):1478), a C-terminal T-Ag fragment T411-708 was not expressed to detectable levels (Fig. 1A and 2A, lane c). cT-Ag and N-terminal T-Ag fragments, but not wtT-Ag, show stable association with the constitutively expressed, cytosolic stress protein Hsp73 (Fig. 2B, lanes a and b) (Schirmbeck, R. and J. Reimann, Eur.J.Immunol. 24 (1994):1478; Schirmbeck, R., W. Bohm, and J. Reimann, Eur.J.Immunol 27 (1997):2016). We have shown stable complex formation of T-Ag variants with Hsp73 in pulse chase experiments (Schirmbeck, R., W. Bohm, and J. Reimann, Eur.J.Immunol. 27 (1997):2016). These data indicated that Hsp73 stabilizes expression of truncated T-Ag fragments. The critical role of the N-terminal Hsp73 binding site of T-Ag in the accumulation of mutant T-Ag variants was confirmed in expression studies using C-terminal T-Ag fragments. The T411-708 T-Ag variant (Fig. 1A; 2A; lane c) and other N-terminally truncated T-Ag variants (data not shown) could not be expressed in transfectants to detectable levels. Cytosolic capture by, and stable binding of cT-Ag or C-terminally (but not N-terminally) truncated T-Ag variants to Hsp73 thus allows their accumulation In cells to easily detectable levels.
Enhanced expression of Hsp73-associated T-Ag mutants was observed in different cell types derived from different species. Transient transfection of TC7 cells (African green monkey kidney cells), L929 cells (murine fibroblasts), Hela cells (human epithelial cells), or LMH cells (chicken hepatoma cells) with the cT-Ag-encoding pCl/cT vector lead to complex formation of cT-Ag with Hsp73 in these cells of different species and tissue origin (data not shown). Hsp73 from fibroblasts, hepatoma, lymphoma, mastocytoma and epithelial cells of human, mouse, chicken and monkey origin thus interacts with mutant T-Ag.
Thus, in eukaryotic transfectants, mutant and truncated T-Ag variants containing an intact N-terminus show stable expression and association with Hsp73. In contrast, TAg variants lacking an intact N-terminus are difficult to express. N-terminal binding of Hsp73 (Hsc70) to T-Ag involves the residues 1-97 of the viral nucleoprotein but the exact binding site is not known (Sawai, E.T., G. Rasmussen, and J.S. Butel, Virus Res. 31 (1994):367). Hsp-binding seems to involve only N-terminal T-Ag sequences because Hsp-binding to T-Ag was affected neither by extensive C-terminal truncations, nor by in frame fusions of different heterologous protein fragments to the N-terminal T-Ag fragment (see below).
The stability of the inaction between Hsp73 and mutant T-Ag variants is unusual. The protein substrate initially bound to Hsp40 is transferred to the ATP-bound state of Hsp73 showing low affinity and fast exchange rate for the substrate. ATP hydrolysis converts Hsp70 into the ADP-bound state with high affinity and a slow exchange rate for the substrate. This rate-limiting step stabilizes the chaperone-peptide interaction (reviewed in Bukau, B. and A.L. Horwich, Cell 92 (1998):351). ATP hydrolysis thus sequesters the substrate protein. Final steps in the ATPase cycle involve the release of ADP and phosphate, the release of substrate, and the binding of ATP. These steps seem to be regulated by chaperones of yet another class of Hsp molecules, the GrpE family. Evidence from pulse chase experiments indicates that the accumulation of large amounts of truncated or chimeric T-Ag/Hsp73 complexes in transfectants results from a slow exchange rate of the mutated T-Ag substrate from Hsp73 (Schirmbeck, R., W. Bohm, and J. Reimann, Eur.J.Immunol. 27.(1997);2018).

### Example 6: Protein fragments are often difficult to express in sukaryotic calls.

In contrast to the N-terminal T-Ag fragment or cT-Ag, many fragments of other proteins are difficult to express in eukaryotic cells. We confirmed this in attempts to construct expression systems for N-terminal or internal fragments that represent potentially immunogenic areas of large microbial proteins. The large surface antigen (LS) of hepatitis B virus (HBV) contains the 163 residue preS domain (composed of the 108 residue preS1 and the 55 residue preS2 domains) and the 224 residue small surface antigen (S) protein (reviewed in Heermann, K.-H. and W.H. Gerlich. 1991. Surface proteins of hepatitis B virus. In A. McLachlan, editor. CRC Press, Boca Raton, Ann Arbor, Boston, London. 109). We cloned the complete LS gene and the N-terminal preS-encoding fragment (Fig. 1B) into eukaryotic expression vectors in which these genes were expressed under HCMV early region promoter control. Expression of the LS antigen (Fig. 3A, lane b) but not of its N-terminal preS fragment (Fig. 3A, lane a) was detectable in Western analyses of lysates of transiently transfected LMH hepatoma cells. Similar data were obtained using other transfected cell lines (data not shown). Whereas the Hsp73-associated T1-272 fragment accumulated to high steady state levels in transiently transfected LMH cells (Fig. 2B, lane b), expression of the LS antigen in these cells was detectable only in ³⁵S-methionine-labeled cells or in Western blots, but not in Coomassie blue stained gels (data not shown). Similar data were obtained in stable transfected cell lines expressing the LS antigen (Fig. 3B). The Hsp73-binding to the N-terminus of truncated T-Ag seems to display unique features that allow its stable expression in eukaryotic cells.
We encountered similar difficulties in attempts to express an internal fragment of the reverse transcriptase (RT) of SIV to detectable levels in eukaryotic transfectants. We used an internal, PCR-amplified 132 residue fragment (residue 281-412) of RT from SIVmac239 (Fig. 1C). We did not succeed in expressing this fragment to detectable levels in different cell lines transiently or stably transfected using different expression constructs (data not shown). Hence, N-terminal and internal fragments of proteins are difficult to express.

### Example 7: C-terminal fusion of heterologous protein fragments to Hsp73-binding T-Ag fragments allows their stable expression.

The Hsp73/T-Ag system was used to facilitate expression of heterologous protein fragments. The 229 residue cytosolic variant cT1-272 representing the N-terminal TAg with a deletion of the nuclear localisation signal (aa 110-152) was generated (Fig. 1D). A cT1-272/preS chimeric gene was constructed by fusing in frame the 163 residue preS domain C-terminally to the cT1-272 antigen (Fig. 1D). Expression of the cT1-272/preS construct in stable RBL5 transfectants revealed abundant steady state levels of the chimeric protein readily detectable in Coomassie Blue-stained gels. Two prominent protein bands of about 45 and 70 kDa size were seen (Fig. 4A, lane a). The 45 kDa protein reacted in Western blot anlyses with the T-Ag-specfic mAb 108 (Fig. 3B, lane a) as well as with the preS1-specific antiserum (Fig. 3D, lane a). The 70 kDa protein reacted with the anti-Hsp70-specific mAb 3A3 (Fig. 3C, lane a). Hsp73 complexed chimeric proteins were coprecipitated by the Hsp73-specific mAb SPA815 (data not shown, Schirmbeck, R., W. Bohm, and J. Reimann. Eur.J.Immunol. 27 (1997):2016). C-terminal fusion to the N-terminal, Hsp73-binding cT1-272 protein hence facilitated expression of the preS domain of the HBV surface antigen to detectable levels.
Similar data were obtained using the cT1-272/RT132 construct generated by fusing the 132 residue SIV RT fragment C-terminally to the cT1-272 protein (Fig.1D). The cT1-272/RT132 chimeric protein was immunprecipitated with the T-Ag specific mAb PAb108 from lysates of transfected cells. Coomassie Blue staining of the gels revealed two prominent protein bands of about 40 and 70 kDa size (Fig. 3A, lane b). Western blot analyses continued that the 40 kDa protein contained the T-Ag (Fig. 3B, lane b) as well as the RT fragment (Fig. 3E, lane b). The RT fragment was detected with a rabbit RT-specific antiserum (see above). Western blotting and Hsp73-specific immunoprecipitation confirmed the Hsp73 association of the chimeric protein (Fig. 3C, lane b; data not shown). These data indicate that an internal protein fragment can be expressed to readily detectable levels by fusing it to an N-terminal T-Ag fragment

### Example 8: Vaccination with plasmid DNA encoding Nsp73.blnding T-Ag variants but not wtT-Ag elicits T-Ag-specific antibody responses.

We compared the murine humoral and cellular immune responses to T-Ag inducible by vaccination with plasmid DNA encoding either Hsp-binding, truncated or chimeric T-Ag variants, or non-Hsp-binding wtT-Ag.
*H-2^{b} mice recognize at least two N-terminal, D^{b}-restricted CTL epitopes present on the cT1-272 fragment_{;} i.e. epitope I (T₂₀₇₋₂₁₅) and epitope II*/*III (T₂₂₃₋₂₃₁)* (Anderson, R.W., M.J. Tevethia, D. Kalderon, A.E. Smith, and S.S. Tevethia, J. Virol. 62 (1988):285; Tanaka, Y. and S.S. Tevethia, J.Immunol. 140 (1988):4348; Tanaka, Y., R.W. Anderson, W.L. Maloy, and S.S. Tevethia, Virology 171 (1989):205; Tevethia, S.S., M. Lewis, Y, Tanaka, J. Milici, B.B. Knowles, W.L. Maloy, and R.W. Anderson, J.Virol, 64 (1990):1192; Deckhut, A.M. and S.S. Tevethia, J.Immunol. 148 (1992):3012; Lippolis, J.D., L.M. Mylin, D.T. Simmons, and S.S. Tevethia, J.Virol. 69 (1995):3134; Mylin, LM., A.M. Deckhut, R.H. Bonneau, T.D. Kierstead, M.J. Tevethia, D.T. Simmons, and S.S. Tevethia, J.Virol. 208 (1995):159). We inoculated 100 µg 'naked' plasmid DNA by a single intramuscular injection into C57BL/6 (H-2^{b}) mice. The plasmid DNA encoded either wtT-Ag (pCl/T), or mutated cT-Ag (pCl/cT), or chimeric cT1-272/preS (pCi/cT1-272/preS). Specific, MHC-I-restricted CD8⁺ CTL reactivity was measured in spleens of vaccinated and control mice 3 weeks post-immunization. As detectable in short-term ⁵¹Cr-release assays against T-Ag-expressing or control targets, all mice vaccinated with one of the three tested constructs displayed high and specific CTL reactivity against T-Ag and cT-Ag that was lacking in control mice (Fig. 5). We could not detect CTL reactivity in H-2^{b} mice specific for preS or RT epitopes in this system (data not shown).
A strikingly different picture emerged when the humoral (serum antibody) response to T-Ag inducible by different variants of the T-Ag through DNA vaccination was compared. DNA-immunized C57BU6 mice were bled 6-12 weeks post-vaccination to test their serum antibody reactivity against T-Ag (Fig. 6, lower panel) and Hsp73 complexed cT-Ag (Fig. 6, upper panel) in Western blots. DNA-based vaccination with wtT-Ag-expressing plasmids did not induce detectable levels of T-Ag-specific serum antibodies (Fig. 6 A; lanes b). This is expected because the SV40 nucleoprotein T-Ag is a strictly intracellular protein. In contrast, vaccination with mutant cT-Ag-expressing or chimeric cT1-272/preS protein-encoding plasmids elicited readily detectable levels of T-Ag-specific serum antibodies (Fig. 6A, lanes c and d). Autoantibodies against Hsp73 were not detectable (Fig. 6A, upper panel). These unexpected findings indicate that Hsp73-associated, mutant endogenous protein antigens, but not non-Hsp73-binding, native endogenous protein antigens can efficiently elicit antibody responses if delivered by DNA vaccination.

### Example 9: DNA vaccination with plasmids encoding Hsp73-binding, chimeric T-Ag elicits efficient antibody responses against the heterologous fusion partner.

Because DNA-based immunization with endogenous mutant but not wild-type T-Ag efficiently elicited serum antibody responses against T-Ag, we tested if chimeric constructs containing an N-terminal T-Ag fragment can be used to elicit antibodies against the protein fragment fused C-terminally to T-Ag. Mice were vaccinated with plasmid DNA encoding either the large surface protein LS of HBV, or its N-terminal preS (preS1 and preS2) domain, or the chimeric cT1-272/preS protein. The antibody response against the preS domains was measured in mice 6-12 weeks post-vaccination in Western blot analyses using yeast-derived HBsAg particles containing antigenic preS epitopes (L*S) and small S protein (Fig. 6B, lane a). Vaccination with DNA of the cTt-272/preS-encoding expression vector efficiently induced preS-specific serum antibodies. The serological reactivity was specific for the preS domain because the antibodies reacted with the large surface protein L*S protein but did not cross-react against the small surface antigen S (Fig. 6B, lane d). Immunization with plasmid DNA encoding only the preS domain that was undetectable in expression analyses (see Fig. 3A) did not elicit preS-specific antibodies (Fig. 6B, lane b). As shown previously, DNA immunization with the LS-encoding plasmid induced serum antibodies reactive with both the large L*S and the small S protein (Fig. 6B, lane c). These data demonstrate that Hsp73-facilitated expression of T-Ag-containing, chimeric protein in DNA vaccination supports the generation of antibody responses against the heterologous fusion partner. In DNA-based vaccination, the system can be used to dissect humoral and cellular immune responses against selected domains, fragments or epitopes of a complex antigen which would be very difficult to express in many instances.

### Example 10:PreS-specific antibody responses are induced by DNA- but not surface particle-based vaccination.

Mice were vaccinated either with 50 µg CHO-derived particles containing L/M/S surface proteins (with/without alum), or with 100 µg pCI/LS plasmid DNA encoding the L surface antigen. Specific antibody responses of immunized mice were tested 12 weeks post-vaccination for reactivity either against the S protein In ELISA (Fig. 3A) or Western (Fig. 3B), or against the preS domain in Western blot analyses using the cT1-272preS antigen (Fig. 3C).
Mice immunized with CHO-derived HBsAg particles developed high antibody titers against particulate S protein (Fig. 3A, lane a). Antibody titers in mice immunized with HBsAg particles formulated in alum adjuvants were 10-20 fold higher than those in mice vaccinated with 'naked' HBsAg particles as previously described (Schirmbeck, R., K. Melber, T. Mertens, and J. Reimann, J.Virol. 68 (1994):1418). These antisera reacted in Western blots against with the S-containing L*S protein and the small S surface protein (Fig. 3B, lane a) but not against the cT1-272preS chimeric protein (Fig. 3C, lane a). Such antisera with S-specific reactivity that lack preS-specific reactivity were found in all BALB/c or C57BL/6 mice immunized with 0.5 - 50 µg CHO-derived surface protein particles (with or without alum) (data not shown). No preS-specific antibody reactivity was detectable in serum samples taken between 3 and 24 weeks post-immunization (data not shown).
Antisera from mice injected with pCI/LS plasmid DNA showed only moderate anti S-protein reactivity (400-900 mlU/ml) detectable in ELISA (Fig. 3A, lane b). Vaccination with pCI/LS plasmid DNA also induced antibodies reactive in Western blots against the S-containing L*S protein and the S protein (Fig. 3B, lane b). This reactivity apparently correlated with anti-S titers measured in ELISA (compare Fig. 3A and B, lanes a and b). In addition, a prominent reactivity against the cT1-272preS antigen was readily detectable in Western blots (Fig. 3C, lane c). Hence, vaccination with plasmid DNA, but not vaccination with surface particles induced antibody responses against preS determinants in mice.

### References

1. Bukau, B. and A.L. Horwich, Cell 92 (1998):351
2. Blachere, N.E., Z. Li, R.Y. Chandawarkar, R. Suto, N.S. Jaikaria, S. Basu, H. Udono, and P.K. Srivastava, J.Exp.Med. 186 (1997):1315
3. Heikema, A., E. Agsteribbe, J. Wilschut, and A. Huckriede, Immunol.Lett. 57 (1997):69
4. Lammert, E., D. Arnold, M. Nijenhuis, F. Momburg, G.J. Hammerling, J. Brunner, S. Stevanovic, H.G. Rammensee, and H. Schild, Eur.J.Immunol. 27 (1997):923
5. Peng, P., A. Menoret, and P.K. Srivastava, J.Immunol.Methods 204 (1997):13
6. Roman, E. and C. Moreno, Immunology 90 (1997):52
7. Nieland, T.J.F., M.C.A. Tan, M.M. van Muijen, F. Koning, A.M. Kruisbeek, and G.M. van Bleek, Proc.Natl.Acad.Sci.USA 93 (1996):6135
8. Roman, E. and C. Moreno, Immunology 88 (1996):487
9. Arnold, D., S. Faath, H.G. Rammensee, and H. Schild, J.Exp.Med. 182 (1995):885
10. Srivastava, P.K., H. Udono, N.E. Blachere, and Z. Li, Immunogenetics 39 (1994):93
11. Udono, H. and P.K. Srivastava, J.Exp.Med. 178 (1993):1391
12. Clupitu, A.-M., M. Petersson, C.L. O'Donnell, K. Williams, S. Jindal, R. Kiessling, and M.J. Welsh, J.Exp.Med. 187 (1998):685
13. Udono, H. and P.K. Srivastava, J.Immunol. 152 (1994):5398
14. Suto, R. and P.K. Srivastava, Science 269 (1995):1585
15. Suzue, K. and R.A. Young, J.Immunol. 156 (1996):873
16. Schirmbeck, R. and J. Reimann, Eur.J.Immunol. 24 (1994):1478
17. Donnelly, J.J., J.B. Ulmer, J.W. Shiver, and M.A. Liu, Annu.Rev.lmmunol. 15 (1997):617
18. Karasuyama, H. and F. Melchers, Eur.J.Immunol. 18 (1988):97
19. Schirmbeck, R., J. Zerrahn, A. Kuhröber, E. Kury, W. Deppert, and J.Reimann, Eur.J.Immunol. 22 (1992):759
20. Schirmbeck, R., W. Deppert, E. Kury, and J. Reimann, Cell.Immunol. 149 (1993):444
21. Schirmbeck, R., J. Zerrahn, A. Kuhröber, W. Deppert, and J. Reimann, Eur.J.Immunol. 23 (1993):1528
22. von Hoyningen-Huene, V., M. Kurth, and W. Deppert, Virology 190 (1992):155
23. Schirmbeck, R., K. Melber, A. Kuhröber, Z.A. Janowicz, and J. Reimann, J.Immunol. 152 (1994):1110
24. Schirmbeck, R., K. Melber, T. Mertens, and J. Reimann, J.Virol. 68 (1994):1418
25. Böhm, W., A. Kuhröber, T. Paier, T. Mertens, J. Reimann, and R. Schirmbeck, J.Immunol.Methods 193 (1996):29
26. Gurney, E.G., S. Tamowsky, and W. Deppert, J.Virol. 57 (1986):1168
27. Craig, E.A., Science 260 (1993):1902
28. Welch, W.J. and J.P. Suhan, J.Cell Biol.103 (1993):2035
29. Schirmbeck, R., W. Bohm, and J. Reimann, Eur.J.Immunol. 27 (1997):2016
30. Heermann, K.-H. and W.H. Gerlich. 1991. Surface proteins of hepatitis B virus. In A. McLachlan, editor. CRC Press, Boca Raton, Ann Arbor, Boston, London. 109
31. Anderson, R.W., M.J. Tevethia, D. Kalderon. A.E. Smith, and S.S. Tevethia, J. Viol. 62 (1988):285
32. Tanaka, Y. and S.S. Tevethia, J.Immunol.140 (1988):4348
33. Tanaka, Y., R.W. Anderson, W.L. Maloy, and S.S. Tevethia,Virology 171 (1989):205
34. Tevethia, S.S., M. Lewis, Y. Tanaka, J. Milici, B.B. Knowles, W.L. Maloy, and R.W. Anderson, J.Virol. 64 (1990):1192
35. Deckhut, A.M. and S.S. Tevethia, J.Immunol. 148 (1992):3012
36. Lippolis, J.D., L.M. Mylin, D.T. Simmons, and S.S. Tevethia, J.Virol. 69 (1995):3134
37. Mylin, L.M., A.M. Deckhut, R.H. Bonneau, T.D. Kierstead, M.J. Tevethia, D.T. Simmons, and S.S. Tevethia, J.Virol. 208 (1995):159
38. Sawai, E.T., G. Rasmussen, and J.S. Butel, Virus Res. 31 (1994):367
39. Dice, J.F. and S.R. Terlecky. 1994. Selective degradation of cytosolic proteins by lysosomes. In Cellular proteolytic systems. A.J. Ciechanover and A.L. Schwartz, editors. WileyLiss, 55-64
40. Hayes, S.A. and J.F. Dice, J.Cell Biol. 132 (1996):255
41. Chiang, H.-L., S.R. Terlecky, C.P. Plant, and J.F. Dice, Science 246 (1989):382
42. Dice, J.F., Trends Biochem.Sci. 15 (1990):305
43. Terlecky, S.R., H.-L. Chiang, T.S. Olson, and J.F. Dice, J.Biol.Chem. 267 (1992):9202
44. Cuervo, A.M. and J.F. Dice, Science 273 (1996):501
45. Dice, J.F., F. Agarraberes, M. Kirven-Brooks, L.J. Terlecky, and S.R. Terlecky. 1994. Heat shock 70-kD proteins and lysosomal proteolysis. In The biology of heat schock proteins and molecular chaperones. R.I. Morimoto, A. Tissieres, and C. Georgopoulos, editors. CSHL Press, Cold Spring Harbor. 137-151

## Claims

1. A polynucleotide encoding a fusion protein which is stable in a cell, said fusion protein comprising:
(a) a first (poly)peptide which as such is unstable in a cell having a half-time of less than two hours; and
(b) a second (poly)peptide which is a viral T-antigen carrying an internal and/or C-terminal deletion and which co-precipitates a chaperone, belonging to the family of heat shock protein (Hsp) 70 chaperones.

2. The polynucleotide of claim 1, wherein said first (poly)peptide is or comprises an epitope and/or a functional and/or structural domain of a protein, and/or a mutated or truncated variant of a protein.

3. The polynucleotide of claim 1, wherein the function and/or structure of the N-terminal J domain of said viral T antigen is maintained.

4. polynucleotide of claim 1 or 3, wherein said viral T antigen is a vital large T-antigen.

5. The polynucleotide of any one of claims 1, 3 and 4, wherein said viral T antigen is SV40 T-antigen. (1) having a half-time of less than two hours (2) which is a viral T-antigen carrying an internal and/or C-terminal deletion and

6. The polynucleotide of claim 5, wherein said viral large T antigen is SV40 large T-antigen.

7. The polynucleotide of claim 6, wherein the about 300 C-terminal amino acids of said SV40 large T antigen are deleted.

8. The polynucleotide of claims 6 or 7, wherein said SV40 large T antigen contains amino acids 1 to 272.

9. The polynucleotide of any one of claims 1 and 3 to 8, wherein the internal deletion comprises at least part of the nuclear localisation signal.

10. The polynucleotide of claim 9, wherein amino acids 110 to 152 are deleted.

11. The polynucleotide of any one of claims 1 to 10 further encoding a tag.

12. The polynucleotide of any one of claims 1 to 11, wherein said first and second (poly)peptide are linked via a protease cleavage site.

13. A vector comprising the polynucleotide of any one of claims 1 to 12.

14. A host cell comprising the polynucleotide of any one of claims 1 to 12, or the vector of claim 13.

15. A method for the production of the fusion protein as defined in any one of claims 1 to 12, said method comprising:
(a) culturing the host cell of claim 14 under conditions that allow the synthesis of said fusion protein; and
(b) recovering said fusion protein from the culture.

16. The method of claim 15 further comprising the step of separating said fusion protein from complexed chaperones.

17. A fusion protein encoded by the polynucleotide of any one of claims 1 to 12, or the vector of claim 13, or obtainable or obtained by the method of claim 15 or 16.

18. A method for the production of a first (poly)peptide as defined in claim 1 or 2, said method comprising:
(a) culturing the host cell of claim 14 under conditions that allow the synthesis of a fusion protein as defined in claim 17;
(b) recovering said fusion protein from the culture; and
(c) separating said second (poly)peptide from said fusion protein by proteolytic cleavage.

19. A method for the production of a complex comprising the fusion protein of claim 17 and a chaperone as defined in claim 1, said method comprising:
(a) culturing the host cell of claim 14 under conditions that allow complex formation of said fusion protein with said chaperone; and
(b) recovering said complex from the culture.

20. A method for the production of an antibody directed against a first (poly)peptide as defined in claim 1 or 2, said method comprising administering in an amount sufficient to induce a humoral immune response the polynucleotide of any one of claims 1 to 12, the vector of claims 13, the fusion protein of claim 17 and/or the complex obtainable or obtained by the method of claim 19 to a non-human subject, wherein said antibody is for use in an immunoassay.

21. A method for the production of an antibody directed against a first (poly)peptide as defined in claim 1 or 2, said method comprising the steps of:
(a) culturing the host cell of claim 14 under conditions that allow the synthesis of a fusion protein as defined in claim 17;
(b) recovering said fusion protein from the culture;
(c) separating said second (poly)peptide from said fusion protein by proteolytic cleavage; and
(d) administering in an amount sufficient to induce a humoral immune response the first (poly)peptide obtained in (c) to a non-human subject, wherein said antibody is for use in an immunoassay.

22. A kit comprising:
(a) the polynucleotide of any one of claims 1 to 12;
(b) the vector of claim 13;
(c) the host cell of claim 14;
(d) the fusion protein of claim 17;
(e) the complex obtainable or obtained by the method of claim 19.

23. A diagnostic composition comprising the polynucleotide of any one of claims 1 to 12, the vector of claim 13, the fusion protein of claim 17, and/or the complex obtainable or obtained by the method of claim 19.

24. An in vitro method for the detection of the presence of an epitope comprised in a (poly)peptide as defined in claim 1 or 2, said method comprising:
(a) contacting the fusion protein of claim 17 with an antibody or a cytotoxic T-lymphocyte (CTL) under conditions that allow binding of said antibody or CTL to said epitope; and
(b) detecting whether the antibody or CTL has bound to said epitope.

25. An in vitro method for the detection of the presence of an epitope comprised in a (poly)peptide as defined in claim 1, said method comprising:
(a) culturing the host cell of claim 14 under conditions that allow the synthesis of a fusion protein as defined in claim 17;
(b) recovering said fusion protein from the culture;
(c) separating said second (poly)peptide from said fusion protein by proteolytic cleavage;
(d) contacting the first (poly)peptide obtained in (c) with an antibody or a cytotoxic T-lymphocyte (CTL) under conditions that allow binding of said antibody or CTL to said epitope; and
(e) detecting whether the antibody or CTL has bound to said epitope.

26. The method of claim 24 or 25, wherein said antibody or CTL is derived from an individual infected with a pathogen.

27. The method of claim 24, 25 or 26, wherein the first (poly)peptide of said fusion protein or said first (poly)peptide obtained in step (c) of claim 25 is derived from a pathogen.

28. A pharmaceutical composition comprising the polynucleotide of any one of claims 1 to 12, the vector of claim 13, the fusion protein of claim 17, and/or the complex obtainable or obtained by the method of claim 19, and, optionally, a pharmaceuticauy acceptable carrier and/or diluent.

29. The pharmaceutical composition of claim 28 which is a vaccine.

30. The pharmaceutical composition of claim 29, wherein said vaccine induces a humoral and/or cellular immune response.

31. Use of the polynucleotide of any one of claims 1 to 12 or the vector of claim 13 for the production of an antibody directed against a first (poly)peptide as defined in claim 1 or 2, wherein said antibody is for use in an immunoassay.

## Patentansprüche

1. Ein Polynucleotid, codierend ein Fusionsprotein, das in einer Zelle stabil ist, wobei das Fusionsprotein umfasst:
(a) ein erstes (Poly)Peptid, das als solches in der Zelle instabil ist, welches es eine Halbwertzeit von weniger als 2 Stunden hat; und
(b) ein zweites (Poly)Peptid, das ein virales T-Antigen ist, das eine interne und/oder C-terminale Deletion aufweist und dass ein Chaperon copräzipitiert, das zur Familie der Hitzeschock-Protein-Chaperone (hsp70) gehört.

2. Das Polynucleotid nach Anspruch 1, wobei das erste (Poly)Peptid ein Epitop und/oder eine funktionelle und/oder strukturelle Domäne eines Proteins und/oder eine mutierte oder verkürzte Variante eines Proteins ist oder umfasst.

3. Das Polynucleotid nach Anspruch 1, wobei die Funktion und/oder Struktur der N-terminalen J-Domäne des viralen T-Antigens aufrechterhalten wird.

4. Das Polynucleotid nach Anspruch 1 oder 3, wobei das virale T -Antigen ein virales großes T -Antigen ist.

5. Das Polynucleotid nach irgendeinem der Ansprüche 1, 3 und 4, wobei das virale T-Antigen SV40 T-Antigen ist.

6. Das Polynucleotid nach Anspruch 5, wobei das virale große T-Antigen das große SV40 T-Antigen ist.

7. Das Polynucleotid nach Anspruch 6, wobei etwa 300 C-terminale Aminosäuren des großen SV40 T-Antigen deletiert sind.

8. Das Polynucleotid nach Anspruch 6 oder 7, wobei das große SV40 T-Antigen die Aminosäuren 1 bis 272 enthält.

9. Das Polynucleotid nach irgendeinem der Ansprüche 1 und 3 bis 8, wobei die interne Deletion zumindest einen Teil des Kernlokalisationssignals umfasst.

10. Das Polynucleotid nach Anspruch 9, wobei die Aminosäuren 110 bis 152 deletiert sind.

11. Das Polynucleotid nach irgendeinem der Ansprüche 1 bis 10, wobei das Polynucleotid ferner eine Markierung (Tag) codiert.

12. Das Polynucleotid nach irgendeinem der Ansprüche 1 bis 11, wobei das erste und zweite (Poly)Peptid über eine Protease-Schnittstelle verbunden sind.

13. Ein Vektor, umfassend das Polynucleotid nach irgendeinem der Ansprüche 1 bis 12.

14. Eine Wirtszelle, umfassend das Polynucleotid nach irgendeinem der Ansprüche 1 bis 12 oder den Vektor nach Anspruch 13.

15. Ein Verfahren zur Herstellung des Fusionsproteins wie in irgendeinem der Ansprüche 1 bis 12 definiert, wobei das Verfahren umfasst:
(a) Züchten der Wirtszelle nach Anspruch 14 unter Bedingungen, die die Synthese des Fusionsproteins erlauben; und
(b) Gewinnen des Fusionsproteins aus der Kultur.

16. Das Verfahren nach Anspruch 15, ferner umfassend den Schritt des Trennens des Fusionsproteins von komplexierten Chaperonen.

17. Ein Fusionsprotein, codiert von dem Polynucleotid nach irgendeinem der Ansprüche 1 bis 12 oder von dem Vektor nach Anspruch 13, oder erhältlich oder erhalten durch das Verfahren nach Anspruch 15 oder 16.

18. Ein Verfahren zur Herstellung eines ersten (Poly)Peptids wie in Anspruch 1 oder 2 definiert, wobei das Verfahren umfasst:
(a) Züchten der Wirtszelle nach Anspruch 14 unter Bedingungen, die die Synthese eines Fusionsproteins wie in Anspruch 17 definiert erlauben;
(b) Gewinnen des Fusionsproteins aus der Kultur; und
(c) Trennen des zweiten (Poly)Peptids vom Fusionsprotein durch proteolytische Spaltung.

19. Ein Verfahren zur Herstellung eines Komplexes umfassend das Fusionsprotein nach Anspruch 17 und ein Chaperon wie in Anspruch 1 definiert, wobei das Verfahren umfasst:
(a) Züchten der Wirtszelle nach Anspruch 14 unter Bedingungen, die die Bildung eines Komplexes des Fusionsproteins mit dem Chaperon erlauben; und
(b) Gewinnen des Komplexes aus der Kultur.

20. Ein Verfahren zur Herstellung eines Antikörpers, der gegen ein erstes (Poly)Peptid wie in Anspruch 1 oder 2 definiert gerichtet ist, wobei das Verfahren das Verabreichen des Polynucleotids nach irgendeinem der Ansprüche 1 bis 12, des Vektors nach Anspruch 13, des Fusionsproteins nach Anspruch 17, und/oder des Komplexes, der mit dem Verfahren nach Anspruch 19 erhältlich ist oder erhalten wurde, an ein nicht-humanes Individuum in einer Menge umfasst, die ausreicht, um eine humorale Immunantwort zu induzieren, wobei der Antikörper für die Verwendung in einem Immunoassay bestimmt ist.

21. Ein Verfahren zur Herstellung eines Antikörpers, der gegen ein erstes (Poly)Peptid wie in Anspruch 1 oder 2 definiert gerichtet ist, wobei das Verfahren die folgenden Schritte umfasst:
(a) Züchten der Wirtszelle nach Anspruch 14 unter Bedingungen, die die Synthese eines wie in Anspruch 17 definierten Fusionsproteins erlauben;
(b) Gewinnen des Fusionsproteins aus der Kultur;
(c) Trennen des zweiten (Poly)Peptids von dem Fusionsprotein durch proteolytische Spaltung; und
(d) Verabreichen des ersten (Poly)Peptids, das in (c) erhalten wurde, an ein nicht-humanes Individuum in einer Menge, die ausreicht, um eine humorale Immunantwort zu induzieren, wobei der Antikörper für eine Verwendung in einem Immunoassay bestimmt ist.

22. Ein Kit umfassend:
(a) das Polynucleotid nach irgendeinem der Ansprüche 1 bis 12;
(b) den Vektor nach Anspruch 13;
(c) die Wirtszelle nach Anspruch 14;
(d) das Fusionsprotein nach Anspruch 17; und/oder
(e) den Komplex, der mit dem Verfahren nach Anspruch 19 erhältlich ist oder erhalten wurde.

23. Eine diagnostische Zusammensetzung, umfassend das Polynucleotid nach irgendeinem der Ansprüche 1 bis 12, den Vektor nach Anspruch 13, das Fusionsprotein nach Anspruch 17 und/oder den Komplex, der mit dem Verfahren nach Anspruch 19 erhältlich ist oder erhalten wurde.

24. Ein in vitro-Verfahren zum Nachweis des Vorliegens eines Epitops, das in einem (Poly)Peptid wie in Anspruch 1 oder 2 definiert umfasst ist, wobei das Verfahren umfasst:
(a) Inkontaktbringen des Fusionsproteins nach Anspruch 17 mit einem Antikörper oder einem cytotoxischen T-Lymphocyten (CTL) unter Bedingungen, die das Binden des Antikörpers oder des CTL an das Epitop erlauben; und
(b) Nachweisen, ob der Antikörper oder der CTL an das Epitop gebunden hat.

25. Ein in vitro-Verfahren zum Nachweis des Vorliegens eines Epitops, das in einem (Poly)Peptid wie in Anspruch 1 oder 2 definiert umfasst ist, wobei das Verfahren umfasst:
(a) Züchten der Wirtszelle nach Anspruch 14 unter Bedingungen, die die Synthese eines Fusionsproteins wie in Anspruch 17 definiert erlauben;
(b) Gewinnung des Fusionsproteins aus der Kultur;
(c) Trennen des zweiten (Poly)Peptids von dem Fusionsprotein durch proteolytische Spaltung;
(d) Inkontaktbringen des ersten (Poly)Peptids, das in (c) erhalten wurde mit einem Antikörper oder einem cytotoxischen T-Lymphocyten (CTL) unter Bedingungen, die das Binden des Antikörpers oder des CTL an das Epitop erlauben; und
(e) Nachweisen, ob der Antikörper oder der CTL an das Epitop gebunden hat.

26. Das Verfahren nach Anspruch 24 oder 25, wobei der Antikörper oder der CTL von einem Individuum abgeleitet ist, das mit einem Pathogen infiziert ist.

27. Das Verfahren nach Anspruch 24, 25 oder 26 wobei das erste (Poly)Peptid des Fusionsproteins oder das erste (Poly)Peptid, das im Schritt (c) von Anspruch 25 erhalten wurde, von einem Pathogen abgeleitet ist.

28. Eine pharmazeutische Zusammensetzung, umfassend das Polynucleotid nach irgendeinem der Ansprüche 1 bis 12, den Vektor nach Anspruch 13, das Fusionsprotein nach Anspruch 17, und/oder den Komplex, der mit dem Verfahren nach Anspruch 19 erhältlich ist oder erhalten wurde, und gegebenenfalls einen pharmazeutisch akzeptablen Träger und/oder ein pharmazeutisch akzeptables Verdünnungsmittel.

29. Die pharmazeutische Zusammensetzung nach Anspruch 28, die ein Impfstoff ist.

30. Die pharmazeutische Zuaammensetzung nach Anspruch 29, wobei der Impfstoff eine humorale und/oder zelluläre Immunantwort induziert.

31. Verwendung des Polynucleotids nach irgendeinem der Ansprüche 1 bis 12 oder des Vektors nach Anspruch 13 für die Herstellung eines Antikörpers, der gegen ein erstes (Poly)Peptid wie in Anspruch 1 oder 2 definiert gerichtet ist, wobei der Antikörper für eine Verwendung in einem Imnunoassay bestimmt ist.

## Revendications

1. Un polynucléotide codant une protéine de fusion qui est stable dans une cellule, ladite protéine de fusion comprenant:
(a) un premier (poly)peptide qui en soi est instable dans une cellule et qui a une demi-vie de moins de deux heures; et
(b) un deuxième (poly)peptide, qui est un antigène T viral portant une délétion interne et/ou de la terminaison C et qui co-précipite un chaperon, ledit chaperon appartenant a la famille des protéines de choc thermique chaperonnes (hsp70).

2. Le polynucléotide selon la revendication 1, dans lequel ledit premier (poly)peptide est ou comprend un épitope et/ou un domaine fonctionnel et/ou structurel d'une protéine, et/ou un variant muté ou tronqué d'une protéine.

3. Le polynucléotide selon la revendication 1, dans lequel la fonction et/ou la structure du domaine J N-terminal dudit antigène T viral est conservée.

4. Le polynucléotide selon la revendication 1 ou 3, dans lequel ledit antigène T viral est un antigène grand T viral.

5. Le polynucléotide selon l'une quelquonque des revendications 1, 3 et 4, dans lequel ledit antigène T viral est l'antigène T du SV40.

6. Le polynucléotide selon la revendication 5, dans lequel ledit antigène grand T viral est l'antigène grand T du S40.

7. Le polynucléotide selon la revendication 6, dans lequel les environ 300 acides aminés C-terminaux dudit antigène grand T du SV40 sont délétés.

8. Le polynucléotide selon la revendication 6 ou 7, dans lequel ledit antigène grand T du SV 40 contient les acides aminés 1 à 272.

9. Le polynucléotide selon l'une quelconque des revendications 1 et 3 à 8, dans lequel la délétion interne comprend au moins une partie du signal de localisation nucléaire.

10. Le polynucléotide selon la revendication 9, dans lequel les acides aminés 110 à 152 sont délétés.

11. Le polynucléotide selon l'une quelconque des revendications 1 à 10 codant en outre pour un marqueur.

12. Le polynucléotide selon l'une quelconque des revendications 1 à 11, dans lequel lesdits premier et deuxième (poly)peptides sont liés via un site de clivage par protéase.

13. Vecteur comprenant le polynucléotide selon l'une quelconque des revendications 1 à 12.

14. Cellule hôte comprenant le polynucléotide selon l'une quelconque des revendications 1 à 12, ou le vecteur selon la revendication 13.

15. Procédé pour la production de la protéine de fusion telle que définie dans l'une quelconque des revendications 1 à 12, ledit procédé comprenant:
(a) de mettre en culture la cellule hôte selon la revendication 14 sous des conditions qui permettent la synthèse de ladite protéine de fusion; et
(b) de récupérer ladite protéine de fusion dans la culture.

16. Le procédé selon la revendication 15 comprenant en outre l'étape de séparer ladite protéine de fusion des chaperons complexés.

17. Un protéine de fusion codée par le polynucléotide selon l'une quelconque des revendications 1 à 12, ou le vecteur selon la revendication 13, ou susceptible d'être obtenue ou obtenue par le procédé de la revendication 15 ou 16.

18. Un procédé pour la production d'un premier (poly)peptide tel que défini dans la revendication 1 ou 2, ledit procédé comprenant:
(a) De mettre en culture la cellule hôte selon la revendication 14 sous des conditions qui permettent la synthèse d'une protéine de fusion telle que définie dans la revendication 17 ;
(b) de récupérer ladite protéine de fusion dans la culture; et
(c) de séparer ledit deuxième (poly)peptide de ladite protéine de fusion par clivage protéolytique.

19. Un procédé pour la production d'un complexe comprenant la protéine de fusion selon la revendication 17 et un chaperon tel que défini dans la revendication 1, ledit procédé comprenant:
(a) de mettre en culture la cellule hôte selon la revendication 14 sous des conditions qui permettent la formation du complexe de ladite protéine de fusion avec ledit chaperon; et
(b) de récupérer ledit complexe dans la culture.

20. Un procédé pour la production d'un anticorps dirigé contre un premier (poly)peptide tel que défini dans la revendication 1 ou 2, ledit procédé comprenant d'administrer dans une quantité suffisante pour induire une réaction d'immunité humorale le polynucléotide selon l'une quelconque des revendications 1 à 12, le vecteur selon la revendication 13, la protéine de fusion selon la revendication 17, et/ou le complexe susceptible d'être obtenu ou obtenu par le procédé selon la revendication 19 à un sujet non-hunnain, dans lequel ledit anticorps est destiné à une utilisation dans un immunoessai.

21. Un procédé pour la production d'un anticorps dirigé contre un premier (poly)peptide tel que défini dans la revendication 1 ou 2, ledit procédé comprenant les étapes de:
(a) de mettre en culture la cellule hôte selon la revendication 14 sous des conditions qui permettent la synthèse d'une protéine de fusion telle que définie dans la revendication 17 ;
(b) de récupérer ladite protéine de fusion dans la culture;
(c) de séparer ledit deuxième (poly)peptide de ladite protéine de fusion par clivage protéolytique; et
(d) d'administrer dans une quantité suffisante pour induire une réaction d'immunité humorale le premier (poly)peptide obtenu en (c) à un sujet non-humain, dans lequel ledit anticorps est destiné à une utilisation dans un immunoessai.

22. Un kit comprenant:
(a) le polynucléotide selon l'une quelconque des revendications 1 à 12 ;
(b) le vecteur selon la revendication 13 ;
(c) la cellule hôte selon la revendication 14 ;
(d) la protéine de fusion selon la revendication 17 ; et/ou
(e) le complexe susceptible d'être obtenu ou obtenu par le procédé selon la revendication 19.

23. Une composition de diagnostic comprenant le polynucléotide selon l'une quelconque des revendications 1 à 12, le vecteur selon la revendication 13, la protéine de fusion selon la revendication 17, et/ou le complexe susceptible d'être obtenu ou obtenu par le procédé selon la revendication 19.

24. Un procédé *in vitro* pour la détection de la présence d'un épitope compris dans un (poly)peptide tel que défini dans la revendication 1 ou 2, ledit procédé comprenant:
(a) de mettre en contact la protéine de fusion selon la revendication 17 avec un anticorps ou un lymphocyte T cytotoxique (CTL ; cytotoxic T-lymphocyte) sous des conditions qui permettent la liaison dudit anticorps ou dudit CTL audit épitope ; et
(b) de détecter si l'anticorps ou le CTL s'est lié audit épitope.

25. Un procédé *in vitro* pour la détection de la présence d'un épitope compris dans un (poly)peptide tel que défini dans la revendication 1 ou 2, ledit procédé comprenant:
(a) de mettre en culture la cellule hôte selon la revendication 14 sous des conditions qui permettent la synthèse d'une protéine de fusion telle que définie dans la revendication 17 ;
(b) de récupérer ladite protéine de fusion dans la culture ;
(c) de séparer ledit deuxième (poly)peptide de ladite protéine de fusion par clivage protéolytique;
(d) de mettre en contact le premier (poly)peptide obtenu à l'étape c) avec un anticorps ou un lymphocyte T cytotoxique (CTL) sous des conditions qui permettent la liaison dudit anticorps ou dudit CTL audit épitope ; et
(e) de détecter si l'anticorps ou le CTL s'est lié audit épitope.

26. Le procédé selon la revendication 24 ou 25, dans lequel ledit anticorps ou ledit CTL est obtenu à partir d'un individu infecté avec un pathogène.

27. Le procédé selon la revendication 24, 25 ou 26, dans lequel le premier (poly)peptide de ladite protéine de fusion ou ledit premier (poly)peptide obtenu à l'étape (c) de la revendication 25 est obtenu à partir d'un pathogène.

28. Une composition pharmaceutique comprenant le polynucléotide selon l'une quelconque des revendications 1 à 12 , le vecteur selon la revendication 13, la protéine de fusion selon la revendication 17, et/ou le complexe susceptible d'être obtenu ou obtenu par le procédé selon la revendication 19, et, facultativement, un support et/ou un diluant pharmaceutiquement acceptable.

29. La composition pharmaceutique selon la revendication 28 qui est un vaccin.

30. La composition pharmaceutique selon la revendication 29, dans laquelle ledit vaccin induit une réaction d'immunité humorale et/ou cellulaire.

31. Utilisation du polynucléotide selon l'une quelconque des revendications 1 à 12 ou du vecteur selon la revendication 13 pour la production d'un anticorps dirigé contre un premier (poly)peptide tel que défini dans la revendication 1 ou 2, dans laquelle ledit anticorps est destiné à une utilisation dans un immunoessai.


### SEQUENCE LISTING

(1) GENERAL INFORMATION:
   (i) APPLICANT:
      (A) NAME: REIMANN, Hansjörg
      (B) STREET: Ringstr. 88
      (C) City: Ulm
      (D) STATE: none
      (E) COUNTRY: Germany
      (F) POSTAL CODE (ZIP): 89081

      (A) NAME: SCHIRMBECK, Reinhold
      (B) STREET: Darszigerstr. 13
      (C) CITY: Krumbach
      (D) STATE: none
      (E) COUNTRY: Germany
      (F) POSTAL CODE (ZIP): 86381
   (ii) TITLE OF INVENTION: Method for the production of (poly)peptides
   (iii) NUMBER OF SEQUENCES: 7
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0. version #1.30 (EPO)
(2) INFORMATION FOR SEQ ID NO: 1:
   (i) SEQUENCE CHARACTERISTICS:
      (A) LENGTH: 5 amine acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE BESCRIPTION: SEQ ID NO: 1:
(2) INFORMATION FOR SEQ ID NO: 2:
   (i) SEQUENCE CHARACTERISTICS:
      (A) LENGTH: 6 amino acids
      (8) TYPE: amino acid
      (C) STRANDEDNESS: single
      (D) TOPOLOGY: linear
   (ii) MOLECULE TYPE : peptide
   (iii) HYPOTHETICAL: YES
   (xi) SEQUENCE DESCRIPTION: SEQ ID NO: 2:
   Lys Asp Asp Asp Asp Lys
   1 5
(2) INFORMATION FOR SEQ ID NO: 3 :
   (i) SEQUENCE CHARACTERISTICS:
      (A) LENGTH: 131 amino acids
      (B) TYPE: amino acid
      (C) STRANDEDNESS: single
      (D) TOPOLOGY; linear
   (ii) MOLECULE TYPE: peptide
   (iii) HYPOTHETICAL: YES
   (xi) SEQUENCE DESCRIPTION: SEQ ID NO: 3;
(2) INFORMATION FOR SEQ NO: 4:
   (i) SEQUENCE CHARACTERISTICS:
      (A) LENGTH: 24 base pairs
      (B) TYPE: nucleic acid
      (C) STRANDEDNESS : single
      (D) TOPOLOGY: linear
   (ii) MOLECULE TYPE: other nucleic acid
      (A) DESCRIPTION: /desc = "oligonucleotide"
      (iii) HYPOTHETICAL: YES
      (xi) SEQUENCE DESCRIPTION: SEQ ID NO: 4: TCGAATGGGG CAGAATCTTT CCAC 24
(2) INFORMATION FOR SEQ ID NO: S:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid (A) DESCRIPTION: /desc = "oligonucleotide"
   (iii) HYPOTHETICAL: YES
   (xi) SEQUENCE DESCRIPTION: SEQ ID NO: 5: CCCTGGGACG CGACTTGATT TCGA 24
(2) INFORMATION FOR SEQ ID NO: 6:
   (i) SEQUENCE CHARACTERISTICS:
      (A) LENGTH: 164 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 6:
   Met Gly Gln Asn Leu Ser Thr Ser Asn Pro Leu Gly Phe Phe Pro Asp
   1               5                   10                  15
   His Gln Leu Asp Pro Ala Phe Arg Ala Asn Thr Ala Asn Pro Asp Trp
               20                  25                  30
   Asp Phe Asn Pro Asn Lys Asp Thr Trp Pro Asp Ala Ala Asn Lys Val
           35                  40                  45
   Gly Ala Gly Ala Phe Gly Leu Gly Phe Thr Pro Pro His Gly Gly Leu
       50                  55                  60
   Leu Gly Trp Ser Pro Gln Ala Gln Gly Ile Leu Gln Thr Leu Pro Ala
   65                  70                  75                  80
   Asn Pro Pro Pro Ala Ser Thr Asn Arg Gln Ser Gly Arg Gln Pro Thr
                   85                  90                  95
   Pro Leu Ser Pro Pro Leu Arg Asn Thr His Pro Gln Ala Met Gln Trp
   100 105 110
   Asn Ser Thr Thr Phe His Gln Thr Leu Gln Asp Pro Arg Val Arg Gly
   115 120 125
   Leu Tyr Phe Pro Ala Gly Gly Ser Ser Ser Gly Thr Val Asn Pro Val
   130 135 140
   Leu Thr Thr Ala Ser Pro Leu Ser Ser Ile Phe Ser Arg Ile Gly Asp
   145 150 155 160
   Pro Ala Leu Asn
(2) INFORMATION FOR SEQ ID NO: 7:
   (i) SEQUENCE CHARACTERISTICS:
      (A) LENGTH: 18 amino acids
      (B) TYPE: amino acid
      (C) STRANDEDNESS: single
      (D) TOPOLOGY: linear
   (ii) MOLECULE TYPE: peptide
   (iii) HYPOTHETICAL: YES
   (xi) SEQUENCE DESCRIPTION: SEQ ID NO: 7:
   Glu Pro Phe Arg Lys Ala Asn Pro Asp Val Thr Leu Val Gln Tyr Met
   1 5 10 15
   Asp Asp